(19) Europäisches Patentamt
European Patent Office
Office européen des brevets

(11) Veröffentlichungsnummer: **0 441 192 A2**

(12) # EUROPÄISCHE PATENTANMELDUNG

(21) Anmeldenummer: **91100932.2**

(22) Anmeldetag: **25.01.91**

(51) Int. Cl.5: **C07K 5/02**, A61K 37/64

(30) Priorität: **07.02.90 DE 4003574**

(43) Veröffentlichungstag der Anmeldung:
**14.08.91 Patentblatt 91/33**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Benz, Günter, Dr.**
**Am Bölkumer Busch 5**
**W-5620 Velbert 15(DE)**
Erfinder: **Henning, Rolf, Dr.**
**Böcklinstrasse 16**
**W-5600 Wuppertal 1(DE)**
Erfinder: **Dressel, Jürgen, Dr.**
**Claudiusweg 1**
**W-5600 Wuppertal 1(DE)**
Erfinder: **Voges, Klaus-Peter, Dr.**
**Schmitteborn 161**
**W-5600 Wuppertal 22(DE)**
Erfinder: **Stasch, Johannes-Peter, Dr.**
**Schneewittchenweg 37**
**W-5600 Wuppertal 1(DE)**
Erfinder: **Knorr, Andreas, Dr.**
**Trillsergraben 10**
**W-4006 Erkrath, Hochdahl(DE)**

(54) **Retroisostere Dipeptide, Verfahren zu ihrer Herstellung und ihre Verwendung als Renininhibitoren in Arzneimitteln.**

(57) Die Erfindung betrifft neue retroisostere Dipeptide der allgemeinen Formel I

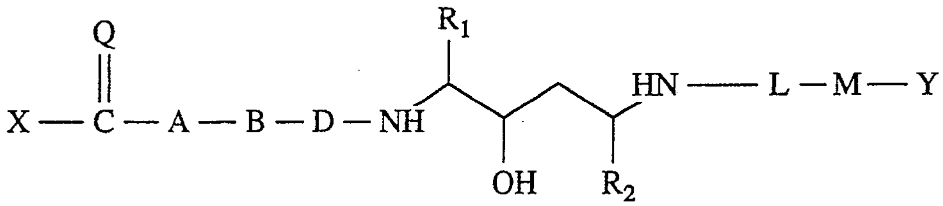

(I)

in welcher X, Q, A, B, D, L, M, Y, $R_1$ und $R_2$ die in der Beschreibung angegebene Bedeutung haben, Verfahren zu ihrer Herstellung und ihre Verwendung als Renininhibitoren in Arzneimitteln, insbesondere in kreislaufbeeinflussenden Arzneimitteln.

EP 0 441 192 A2

# NEUE DIPEPTIDE, VERFAHREN ZU IHRER HERSTELLUNG UND IHRE VERWENDUNG ALS RENININHIBITOREN IN ARZNEIMITTELN

Die Erfindung betrifft neue retroisostere Dipeptide, Verfahren zu ihrer Herstellung und ihre Verwendung als Renininhibitoren in Arzneimitteln, insbesondere in kreislaufbeeinflussenden Arzneimitteln.

Renin ist ein proteolytisches Enzym, das überwiegend von den Nieren produziert und ins Plasma sezerniert wird. Es ist bekannt, daß Renin in vivo vom Angiotensinogen das Dekapeptid Angiotensin I abspaltet. Angiotensin I wiederum wird in der Lunge, den Nieren oder anderen Geweben zu dem blutdruckwirksamen Oktapeptid Angiotensin II abgebaut. Die verschiedenen Effekte des Angiotensin II wie Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Die Aktivität des Renin-Angiotensin-Systems kann durch die Hemmung der Aktivität von Renin oder dem Angiotensin-Konversionsenzym (ACE) sowie durch Blockade von Angiotensin II-Rezeptoren pharmakologisch manipuliert werden. Die Entwicklung von oral einsetzbaren ACE-Hemmern hat somit zu neuen Antihypertensiva geführt (vgl. DOS 3 628 650, Am. J. Med. 77, 690, 1984). ACE wirkt jedoch auch auf andere Substrate als Angiotensin I wie z.B. Kinine ein. Diese können unerwünschte Nebeneffekte wie Prostaglandinfreisetzung und eine Reihe verhaltens- und neurologischer Effekte hervorrufen. Die ACE-Inhibition führt darüberhinaus zu einer Akkumulation von Angiotensin I.

Ein spezifischer Ansatz ist, in die Renin-Angiotensin-Kaskade zu einem früheren Zeitpunkt einzugreifen, nämlich durch Inhibition der sauren Aspartase Renin, die nur Angiotensinogen als Substrat erkennt.

Bisher wurden verschiedene Arten von Renininhibitoren entwickelt: Reninspezifische Antikörper, Phospholipide, Peptide mit der N-terminalen Sequenz des Prorenins, synthetische Peptide als Substratanaloga und modifizierte Peptide. Bei vielen Renininhibitoren wird ferner das Leu-Val-Dipeptid durch Statin (EP 077 029) oder durch isostere Dipeptide ersetzt (vgl. US 442 42 07).

Außerdem werden in der PCT WO 88/02374 Renininhibitoren umfaßt, die als proteasestabilen zentralen Mittelteil retroisostere Dipeptideinheiten enthalten. Retroisostere Dipeptide besitzen eine kopfständige Aminogruppe; die Kupplung zu C-terminalen Aminosäuren führt zu einer Umkehrung der Amidfunktion (Retroamid), die sich durch eine hohe metabolische Stabilität gegenüber enzymatischen Abbau auszeichnet.

Über das erfindungsgemäße Verfahren wurden neue Renininhibitoren gefunden, die überraschenderweise eine hohe Selektivität gegenüber humanem Renin und eine gute orale Wirksamkeit besitzen.

Die Erfindung betrifft retroisostere acylierte Dipeptide der allgemeinen Formel (I)

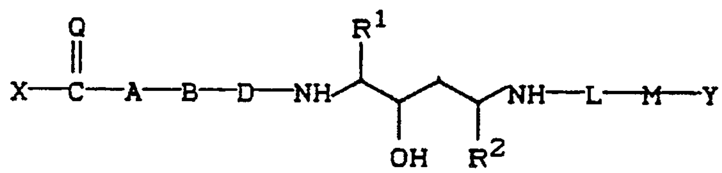
(I)

in welcher

X - für Indolyl, tert.-Butoxy, Morpholino oder für eine Gruppe der Formel

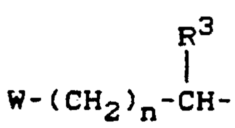 , $R^4$-NH- , 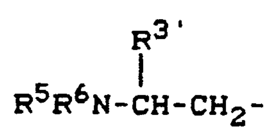

oder für T steht,

worin

$R^3$ und $R^{3'}$ gleich oder verschieden sind und geradkettiges oder verzweigtes Alkyl mit bis zu 8 Kohlenstoffatomen bedeuten, das gegebenenfalls durch Aryl mit 6 bis 10 Kohlenstoffatomen substituiert ist,

n - eine Zahl 1, 2, 3 oder 4 bedeutet,

W - eine Gruppe der Formel
$R^7$-CO-, $R^8$-$SO_2$- oder

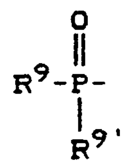

bedeutet,
worin

$R^7$ und $R^8$ gleich oder verschieden sind und geradkettiges oder verzweigtes Alkyl mit bis zu 8 Kohlenstoffatomen oder einen 6-gliedrigen Heterocyclus mit bis zu 3 Heteroatomen aus der Reihe Stickstoff, Sauerstoff oder Schwefel bedeuten,

$R^9$ und $R^{9'}$ gleich oder verschieden sind und Hydroxy oder Alkoxy mit bis zu 8 Kohlenstoffatomen bedeuten,

$R^4$ - einen Kohlenhydratrest mit 4 bis 8 Kohlenstoffatomen bedeutet, wobei die OH-Funktionen des Zuckers gegebenenfalls geschützt sind,

Q - Sauerstoff oder Schwefel bedeutet,

$R^5$ und $R^6$ gleich oder verschieden sind und Wasserstoff, geradkettiges oder verzweigtes Alkyl mit bis zu 8 Kohlenstoffatomen, Phenyl oder eine Aminoschutzgruppe bedeuten,

T - geradkettiges oder verzweigtes Alkenyl mit bis zu 8 Kohlenstoffatomen bedeutet, das gegebenenfalls durch Aryl mit 6 bis 10 Kohlenstoffatomen substituiert ist, das seinerseits durch Halogen, Hydroxy oder Alkoxy mit bis zu 6 Kohlenstoffatomen substituiert sein kann,

A, B und D gleich oder verschieden sind und
- für eine direkte Bindung oder
- für einen Rest der Formel

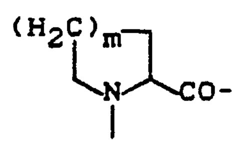

stehen
worin
m - die Zahl 1 oder 2 bedeutet,
oder
- für eine Gruppe der Formel

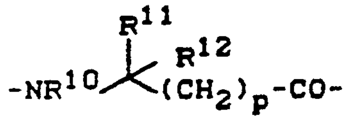

stehen
worin
p - die Zahl 0 oder 1 bedeutet,

$R^{10}$ Wasserstoff, geradkettiges oder verzweigtes Alkyl mit bis zu 6 Kohlenstoffatomen oder eine Aminoschutzgruppe bedeutet,

$R^{11}$ und $R^{12}$ gleich oder verschieden sind und
- Wasserstoff oder Cycloalkyl mit 3 bis 8 Kohlenstoffatomen bedeuten, oder
- einen 3- bis 8-gliedrigen Heterocyclus mit bis zu 4 Heteroatomen aus der Reihe Stickstoff, Sauerstoff oder Schwefel bedeuten,

- geradkettiges oder verzweigtes Alkyl mit bis zu 8 Kohlenstoffatomen bedeuten, das gegebenenfalls durch Alkylthio mit bis zu 6 Kohlenstoffatomen, Hydroxy, Mercapto, Guanidyl oder durch eine Gruppe der Formel $-NR^5R^6$ oder $R^{13}$-OC- substituiert ist,

worin

$R^5$ und $R^6$ die oben angegebene Bedeutung haben

und

$R^{13}$ Hydroxy, Benzyloxy, Alkoxy mit bis zu 6 Kohlenstoffatomen oder die oben aufgeführte Gruppe $-NR^5R^6$ bedeutet, oder das gegebenenfalls durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^5R^6$ substituiert sein kann,

worin

$R^5$ und $R^6$ die oben angegebene Bedeutung haben oder das gegebenenfalls durch einen 5- oder 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

L und M gleich oder verschieden sind und

- für eine direkte Bindung oder

- für eine Gruppe der Formel

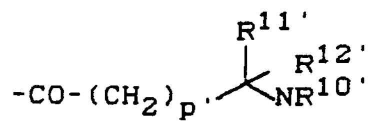

stehen

worin

p', $R^{10'}$, $R^{11'}$ und $R^{12'}$ die oben angegebene Bedeutung von p, $R^{10}$, $R^{11}$ und $R^{12}$ haben,

in ihrer D- oder L-Form oder als D,L-Isomerengemisch,

$R^1$ und $R^2$ gleich oder verschieden sind und

- für geradkettiges oder verzweigtes Alkyl mit bis zu 8 Kohlenstoffatomen stehen, das gegebenenfalls durch Cycloalkyl mit 3 bis 8 Kohlenstoffatomen oder Aryl mit 6 bis 10 Kohlenstoffatomen substituiert ist,

Y - für eine Gruppe der Formel

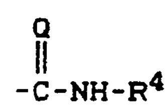

oder $-CO-R^{14}$ steht,

worin

Q und $R^4$ die oben angegebene Bedeutung haben,

$R^{14}$ geradkettiges oder verzweigtes Alkyl mit bis zu 8 Kohlenstoffatomen bedeutet, das gegebenenfalls durch Pyridyl, Phenyl oder die Gruppe $-NR^{15}R^{16}$ substituiert ist,

worin

$R^{15}$ und $R^{16}$ entweder die oben angegebene Bedeutung von $R^5$ und $R^6$ haben und mit dieser gleich oder verschieden sind oder $R^{15}$ Wasserstoff bedeutet und $R^{16}$ die Gruppe der Formel

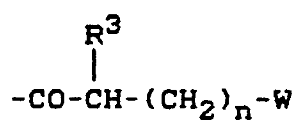

oder

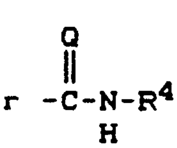

bedeutet

worin

W, Q, n, $R^3$ und $R^4$ die oben angegebene Bedeutung haben

und deren physiologisch unbedenklichen Salze, mit der Maßgabe, daß X nur dann tert.-Butoxy bedeuten darf, wenn Y für die Gruppe

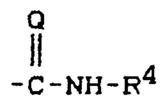 oder 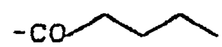

steht.

Als OH-Schutzgruppen eignen sich die üblichen Schutzgruppen wie Methylbenzoyl, Benzoyl, Acetyl oder Benzyl. Bevorzugt sind Benzyl oder Acetyl. Besonders bevorzugt ist Acetyl (Ac).

Aminoschutzgruppe steht im Rahmen der Erfindung für die üblichen in der Peptid-Chemie verwendeten Aminoschutzgruppen.

Hierzu gehören bevorzugt; Benzyloxycarbonyl, 4-Brombenzyloxycarbonyl, 2-Chlorbenzyloxycarbonyl, 3-Chlorbenzyloxycarbonyl, 3,5-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, 3,4,5-Trimethoxybenzyloxycarbonyl, Ethoxycarbonyl, Propoxycarbonyl, Isopropoxycarbonyl, tert-Butoxycarbonyl, Pentoxycarbonyl, Isopentoxycarbonyl, Cyclohexoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis-(4-methoxyphenyl)methoxycarbonyl, Phenacyloxycarbonyl, 2-Trimethylsilylethoxycarbonyl, 2-Triphenylsilylethoxycarbonyl, Menthyloxycarbonyl, Vinyloxycarbonyl, Allyloxycarbonyl, Fluorenyl-9-methoxycarbonyl, Ethylthiocarbonyl, Methylthiocarbonyl, Bintylthiocarbonyl, Tert.-Butylthiocarbonyl, Benzylthiocarbonyl, Formyl, Acetyl, Propionyl, Pivaloyl, 2-Chloracetyl, 2-Bromacetyl, 2-Iodacetyl, 2,2,2-Trifluoracetyl, 2,2,2-Trichloracetyl, Benzoyl, 4-Chlorbenzoyl, 4-Methoxybenzoyl, 4-Nitrobenzyl, 4-Nitrobenzoyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)phosphoryl, Phenoxyphenylphosphoryl, Diethylphosphinyl, Diphenylphosphinyl oder Phthaloyl.

Besonders bevorzugte Aminoschutzgruppen sind Benzyloxycarbonyl, 3,5-Dimethoxybenzyloxycarbonyl, 4-Methoxybenzyloxycarbonyl, 4-Nitrobenzyloxycarbonyl, 2-Nitrobenzyloxycarbonyl, 3,4,5-Trimethoxybenzyloxycarbonyl, Ethoxycarbonyl, Propoxycarbonyl, Isopropoxycarbonyl, tert-Butoxycarbonyl, Cyclohexoxycarbonyl, 2-Chlorethoxycarbonyl, Phenoxyacetyl, Naphthylcarbonyl, Adamantylcarbonyl, Phthaloyl, 2,2,2-Trichlorethoxycarbonyl, 2,2,2-Trichlor-tert-butoxycarbonyl, Menthyloxycarbonyl, Vinyloxycarbonyl, Allyloxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Isovaleroyl oder Benzyloxymethyl.

Die erfindungsgemäßen Verbindungen der allgemeinen Formel (I), haben mehrere asymmetrische Kohlenstoffatome. Sie können unabhängig voneinander in der D- oder der L-Form vorliegen. Die Erfindung umfaßt die optischen Antipoden ebenso wie die Isomerengemische oder Racemate. Bevorzugt liegen die Gruppen A, B, D, L und M unabhängig voneinander in der optisch reinen, bevorzugt in der L-Form vor.

Die Gruppe der Formel

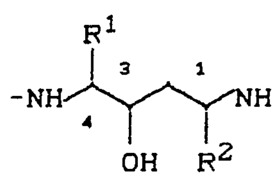

besitzt 3 asymmetrische Kohlenstoffatome (1,3,4), die unabhängig voneinander in der R- oder S-Konfiguration vorliegen können. Bevorzugt liegt diese Gruppe in der 1R, 3S, 4S- Konfiguration , 1R, 3R, 4S-Konfiguration, 1S, 3R, 4S- Konfiguration oder in der 1S, 3S, 4S-Konfiguration vor.

Besonders bevorzugt sind die 1S, 3S, 4S-Konfiguration und die 1R, 3S, 4S-Konfiguration, die in Abhängigkeit der Natur des Substituenten $R^2$ die Konfiguration eines L,L- Dipeptides widerspiegeln.

Die erfindungsgemäßen Verbindungen der allgemeinen Formel (I) können in Form ihrer Salze vorliegen. Dies können Salze der erfindungsgemäßen Verbindungen mit anorganischen oder organischen Säuren oder

Basen sein, Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, Oxalsäure, Glykolsäure, Bernsteinsäure, Maleinsäure, Hydroxymaleinsäure, Methylmaleinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure, Nicotinsäure, Isonicotinsäure, oder Sulfonsäuren wie Methansulfonsäure, Ethansulfonsäure, Benzolsulfonsäure, Toluolsulfonsäure, Naphthalinsulfonsäuren.

Bevorzugt sind Verbindungen der allgemeinen Formel (I),
in welcher
X - für Indolyl, tert`-Butoxy, Morpholino oder für eine Gruppe der Formel

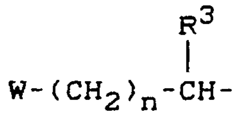 , 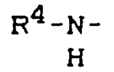 , 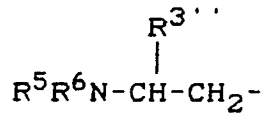

oder für T steht,
worin
$R^3$ und $R^{3'}$ gleich oder verschieden sind und geradkettiges oder verzweigtes Alkyl mit bis zu 6 Kohlenstoffatomen bedeuten, das gegebenenfalls durch Phenyl oder Naphthyl substituiert ist,
n - eine Zahl 1, 2 oder 3 bedeutet,
W - eine Gruppe der Formel
$R^7$-CO-, $R^8$-$SO_2$- oder

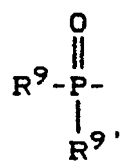

bedeutet
worin
$R^7$ und $R^8$ gleich oder verschieden sind und geradkettiges oder verzweigtes Alkyl mit bis zu 6 Kohlenstoffatomen oder Morpholino bedeuten,
$R^9$ und $R^{9'}$ gleich oder verschieden sind und Hydroxy oder Alkoxy mit bis zu 6 Kohlenstoffatomen bedeuten,
$R^4$ einen Pyranosylrest bedeutet, wobei die OH-Funktionen des Zuckers gegebenenfalls geschützt wird,
Q - Sauerstoff oder Schwefel bedeutet,
$R^5$ und $R^6$ gleich oder verschieden sind und Wasserstoff, geradkettiges oder verzweigtes Alkyl mit bis zu 6 Kohlenstoffatomen oder eine Aminoschutzgruppe bedeuten,
T - geradkettiges oder verzweigtes Alkenyl mit bis zu 6 Kohlenstoffatomen bedeutet, das gegebenenfalls durch Phenyl substituiert ist, das seinerseits durch Fluor, Chlor, Hydroxy oder Alkoxy mit bis zu 4 Kohlenstoffatomen substituiert ist,
A, B und D gleich oder verschieden sind und
- für eine direkte Bindung oder
- für Prolin stehen, oder
- für eine Gruppe der Formel

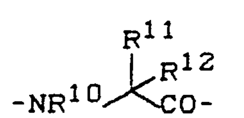

stehen
worin

$R^{10}$ Wasserstoff oder geradkettiges oder verzweigtes Alkyl mit bis zu 4 Kohlenstoffatomen bedeutet,

$R^{11}$ und $R^{12}$ gleich oder verschieden sind und Cyclopentyl, Cyclohexyl, Wasserstoff oder geradkettiges oder verzweigtes Alkyl mit bis zu 6 Kohlenstoffatomen bedeuten, das gegebenenfalls durch Naphthyl oder Phenyl substituiert ist, die ihrerseits durch Fluor, Chlor, Nitro oder Alkoxy mit bis zu 6 Kohlenstoffatomen substituiert sein können,
oder durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe substituiert sind,

L und M gleich oder verschieden sind und
- für eine direkte Bindung oder
- für eine Gruppe der Formel

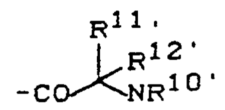

stehen
worin

$R^{10'}$, $R^{11'}$ und $R^{12'}$ die oben angegebene Bedeutung von $R^{10}$, $R^{11}$ und $R^{12}$ haben und mit dieser gleich oder verschieden sind,

in ihrer D- oder L-Form, oder als D,L-Isomerengemisch,

$R^1$ und $R^2$ gleich oder verschieden sind und für geradkettiges oder verzweigtes Alkyl mit bis zu 6 Kohlenstoffatomen stehen, das gegebenenfalls durch Cyclopropyl, Cyclopentyl, Cyclohexyl oder Phenyl substituiert ist,

Y - für eine Gruppe der Formel

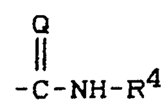

oder -CO-$R^{14}$ steht,
worin

Q und $R^4$ die oben angegebene Bedeutung haben,

$R^{14}$ geradkettiges oder verzweigtes Alkyl mit bis zu 6 Kohlenstoffatomen bedeutet, das gegebenenfalls durch Pyridyl, Phenyl oder durch die Gruppe -$NR^{15}R^{16}$ substituiert ist,
worin

$R^{15}$ und $R^{16}$ entweder gleich oder verschieden sind und die oben angegebene Bedeutung von $R^5$ und $R^6$ haben und mit dieser gleich oder verschieden sind, oder

$R^{15}$ Wasserstoff bedeutet und

$R^{16}$ für eine Gruppe der Formel

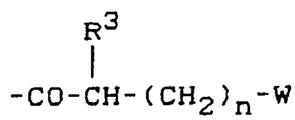

oder

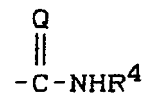

steht,
worin
U, Q, n, $R^3$ und $R^4$ die oben angegebene Bedeutung haben

und deren physiologisch unbedenklichen Salze, mit der Maßgabe, daß X nur dann tert.-Butoxy bedeuten darf, wenn Y für die Gruppe

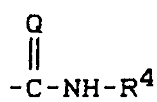 oder 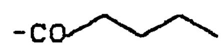

steht.

Besonders bevorzugt sind Verbindungen der allgemeinen Formel (I), in welcher
X - für Indolyl, tert.-Butoxy, Morpholino oder für eine Gruppe der Formel

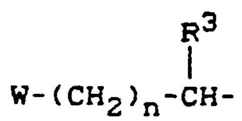 ,

$R^4$-NH- ,

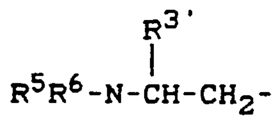

oder für T steht,
worin
$R^3$ und $R^{3'}$ gleich oder verschieden sind und geradkettiges oder verzweigtes Alkyl mit bis zu 4 Kohlenstoffatomen bedeuten, das gegebenenfalls durch Phenyl oder Naphthyl substituiert ist,
n - eine Zahl 1 oder 2 bedeutet,
W - eine Gruppe der Formel $R^7$-CO- , $R^8$-$SO_2$- oder

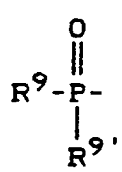

bedeutet,
worin
$R^7$ und $R^8$ gleich oder verschieden sind und geradkettiges oder verzweigtes Alkyl mit bis zu 4 Kohlenstoffatomen oder Morpholino bedeuten,
$R^9$ und $R^{9'}$ gleich oder verschieden sind und Hydroxy oder Alkoxy mit bis zu 4 Kohlenstoffatomen bedeuten,
$R^4$ - einen Rest der Formel

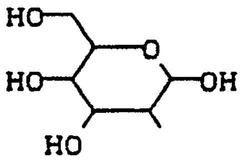, 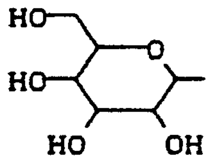, 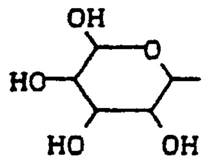 oder 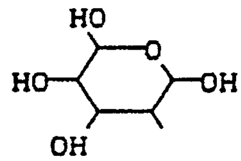

bedeutet

worin die OH-Funktionen gegebenenfalls durch Acetyl geschützt sind

Q - Sauerstoff oder Schwefel bedeutet,

$R^5$ und $R^6$ gleich oder verschieden sind und Wasserstoff, geradkettiges oder verzweigtes Alkyl mit bis zu 4 Kohlenstoffatomen oder eine Aminoschutzgruppe bedeuten,

T - den Rest der Formel

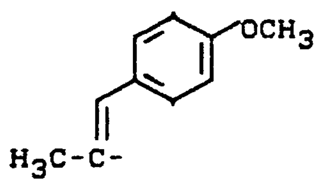

bedeutet,

A, B und D gleich oder verschieden sind und

- für eine direkte Bindung oder
- für Prolin stehen, oder
- für eine Gruppe der Formel

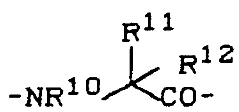

stehen,

worin

$R^{10}$ Wasserstoff oder Methyl bedeutet,

$R^{11}$ und $R^{12}$ gleich oder verschieden sind und Cyclopentyl oder geradkettiges oder verzweigtes Alkyl mit bis zu 4 Kohlenstoffatomen bedeuten, das gegebenenfalls durch Naphthyl oder Phenyl substituiert ist, die ihrerseits durch Fluor, Chlor oder Alkoxy mit bis zu 4 Kohlenstoffatomen substituiert sein können, oder

Alkyl durch Imidazolyl, Triazolyl, Pyridyl oder Pyrazolyl substituiert ist, wobei die -NH-Funktionen gegebenenfalls durch Methyl, Boc oder BOM geschützt sind,

L und M gleich oder verschieden sind und

- für eine direkte Bindung oder
- für eine Gruppe der Formel

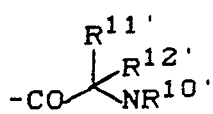

stehen,

worin

$R^{10'}$, $R^{11'}$ und $R^{12'}$ die oben angegebene Bedeutung von $R^{10}$, $R^{11}$ und $R^{12}$ haben und mit dieser gleich oder verschieden sind,

in ihrer D- oder L-Form, oder als D,L-Isomerengemisch,

$R^1$ und $R^2$ gleich oder verschieden sind und

- für geradkettiges oder verzweigtes Alkyl mit bis zu 4 Kohlenstoffatomen stehen, das gegebenenfalls durch Cyclohexyl oder Phenyl substituiert ist,

Y - für eine Gruppe der Formel

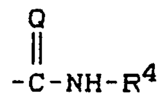

oder -CO-$R^{14}$ steht,

worin

Q und $R^4$ die oben angegebene Bedeutung haben,

$R^{14}$ geradkettiges oder verzweigtes Alkyl mit bis zu 4 Kohlenstoffatomen bedeutet, das gegebenenfalls durch Pyridyl, Phenyl oder durch die Gruppe -$NR^{15}R^{16}$ substituiert ist,

worin

$R^{15}$ Wasserstoff bedeutet und

$R^{16}$ eine Gruppe der Formel

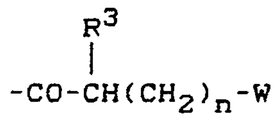

oder

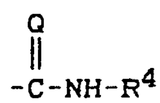

bedeutet, worin

W, Q, n, $R^3$ und $R^4$ die oben angegebene Bedeutung haben

und deren physiologisch unbedenklichen Salze, mit der Maßgabe, daß X nur dann tert.-Butoxy bedeuten darf, wenn Y für die Gruppe

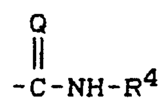 oder 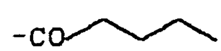

steht.

Außerdem wurde ein Verfahren zur Herstellung der erfindungsgemäßen Verbindungen der allgemeinen Formel (I)

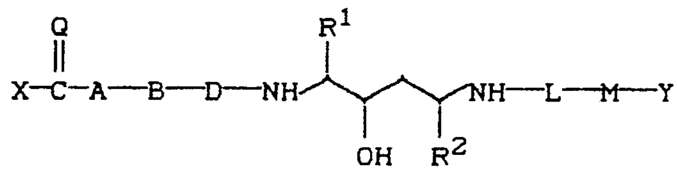 (I)

in welcher

X, A, B, D, $R^1$, $R^2$, L, M, Q und Y die oben angegebene Bedeutung haben,

gefunden, dadurch gekennzeichnet, daß man

Verbindungen der allgemeinen Formel (II)

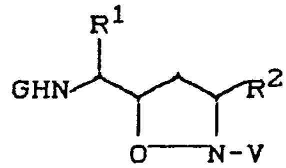

(II)

in welcher
$R^1$ und $R^2$ die oben angegebene Bedeutung haben,
G - für eine der oben aufgeführten Aminoschutzgruppen steht,
und
V - für einen hydrogenolytisch abspaltbaren Rest, wie beispielsweise Benzyl steht,
zunächst durch Hydrogenolyse unter Öffnung des Isoxazolidinrings zu den Aminoalkoholen der allgemeinen Formel (III)

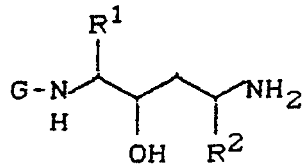

(III)

worin
G, $R^1$ und $R^2$ die oben angegebene Bedeutung haben,
reduziert, gegebenenfalls anschließend mit Verbindungen der allgemeinen Formel (IV) oder (IVa)

HO—L'—M'—Y (IV) HO-Y (IVa)

in welcher Y
die oben angegebene Bedeutung hat
und
L' und M' die oben angegebene Bedeutung von L und M haben, aber nicht gleichzeitig für eine direkte Bindung stehen,
in inerten Lösemitteln, gegebenenfalls in Anwesenheit eines wasserentziehenden Hilfsstoffes und/oder einer Base kondensiert,
anschließend nach Abspaltung der Schutzgruppe G nach bekannter Methode mit Verbindungen der allgemeinen Formel (V)

G'—B'—D'—OH (V)

in welcher
B' und D' die oben angegebene Bedeutung von B und D haben, aber nicht gleichzeitig für eine direkte Bindung stehen
und
G' die oben angegebene Bedeutung von G hat und mit dieser gleich oder verschieden ist,

umsetzt und in einem letzten Schritt nach Abspaltung der Schutzgruppe G' mit Verbindungen der Formel (VI)

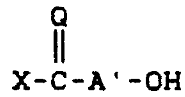

(VI)

in welcher
X und Q die oben angegebene Bedeutung haben
und
A' die oben angegebene Bedeutung von A hat, aber nicht für eine direkte Bindung steht, gegebenenfalls in Anwesenheit einer Base umsetzt.

Das erfindungsgemäße Verfahren kann durch folgendes Formelschema beispielhaft erläutert werden:

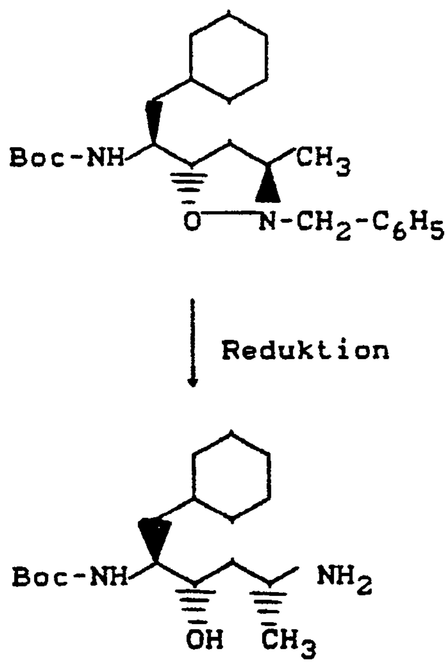

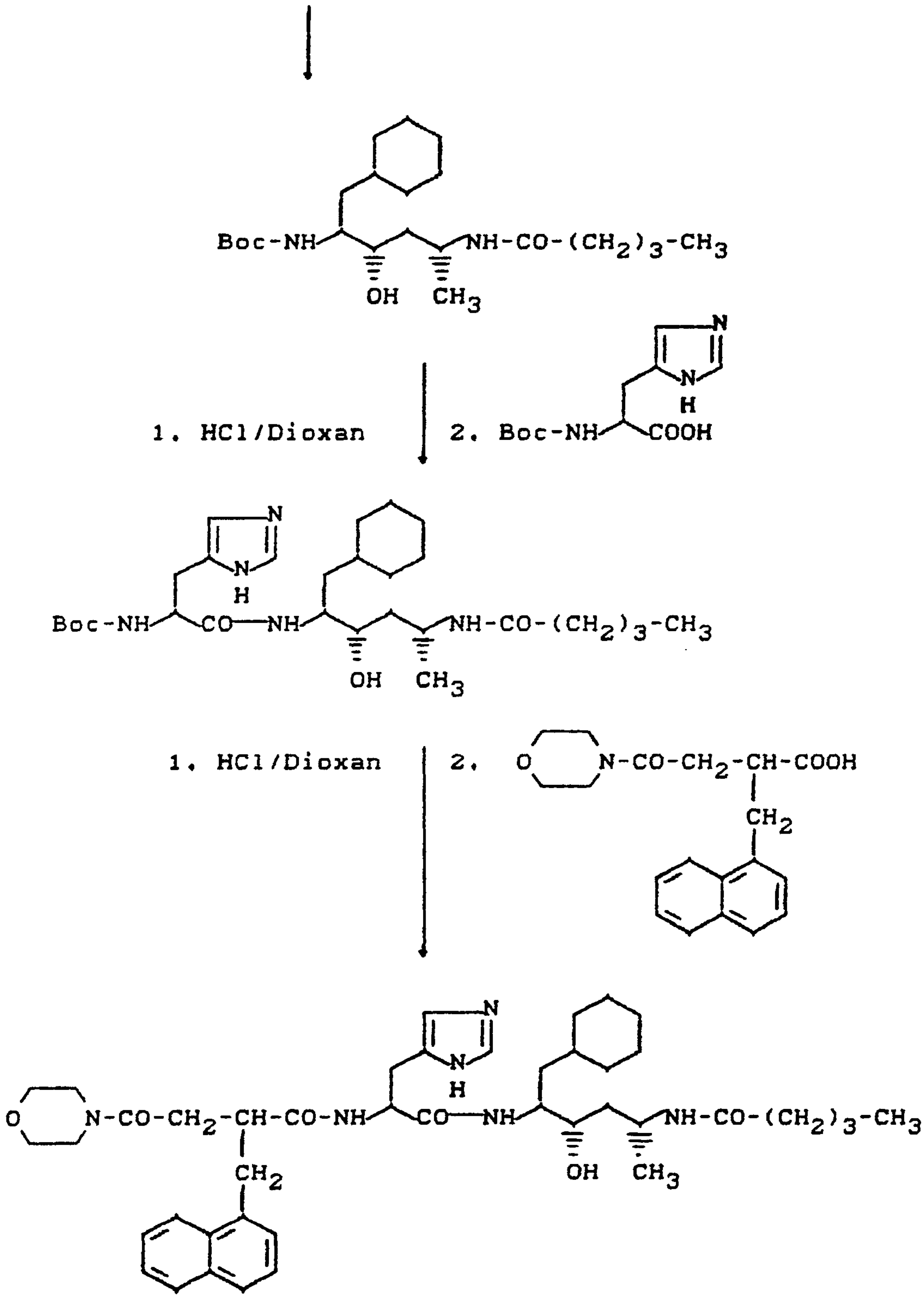


Als Lösemittel eignen sich für alle Verfahrensschritte die üblichen inerten Lösemittel, die sich unter den Reaktionsbedingungen nicht verändern. Hierzu gehören bevorzugt organische Lösemittel wie Methanol, Ethanol, Propanol, Isopropanol oder Ether wie Diethylether, Glykolmono- oder dimethylether, Dioxan oder Tetrahydrofuran, oder Kohlenstoffe wie Benzol, Toluol, Xylol, Cyclohexan oder Erdölfraktionen oder Halogenkohlenwasserstoffe wie Methylenchlorid, Chloroform, Tetrachlorkohlenwasserstoff, oder Aceton, Dimethylsulfoxid, Dimethylformamid, Hexamethylphosphorsäuretriamid, Essigester, Pyridin, Triethylamin oder Picoline. Ebenso ist es möglich, Gemische der genannten Lösemittel zu verwenden.

Besonders bevorzugt sind für die Reduktion Methanol und Essigsäureethylester, für die Peptidkupplung und

die Umsetzung mit Verbindungen der allgemeinen Formeln (IV), (IVa) und (VI) Methylenchlorid.

Die Reduktion der Verbindungen der allgemeinen Formel (II) erfolgt entweder mit den üblichen Katalysatoren, wie beispielsweise Palladiumhydroxid oder Palladium/Kohlenstoff, vorzugsweise mit Palladium/Kohlenstoff oder über eine katalytische Transferhydrierung in an sich bekannter Weise [vgl. Tetrahedron 41, 3469 (1985), 3463 (1985), Synthesis 1987, 531.

Der Katalysator wird in einer Menge von 0,05 bis 1,0 mol, bevorzugt von 0,1 bis 0,5 mol, bezogen auf 1 Mol der Verbindung der allgemeinen Formel (II) eingesetzt.

Die Reduktion wird in einem Temperaturbereich von 40° C bis 160° C, vorzugsweise von 60° C bis 80° C durchgeführt.

Die Reduktion kann sowohl bei Normaldruck als auch bei erhöhtem oder erniedrigtem Druck (beispielsweise 0,5 bis 5 bar), vorzugsweise bei Normaldruck durchgeführt werden.

Die Verbindungen der allgemeinen Formel (II) sind neu und können hergestellt werden, indem man Verbindungen der allgemeinen Formel (VII)

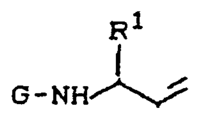

(VII)

in welcher
G die oben angegebene Bedeutung hat,
in einer Cycloadditionsreaktion mit Verbindungen der allgemeinen Formel (VIII)

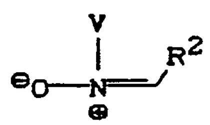

(VIII)

in welcher
V und $R^2$ die oben angegebene Bedeutung haben,
gegebenenfalls in Gegenwart von inerten organischen Lösungsmitteln umsetzt.

Als Lösemittel eignen sich die üblichen organischen Lösemittel, die sich unter den Reaktionsbedingungen nicht verändern. Hierzu gehören bevorzugt Alkohole wie Methanol, Ethanol, Propanol, Isopropanol, n-Butanol oder Ether wie Diethylether, Dioxan, Tetrahydrofuran, Glykolmono- oder -diethylether oder Kohlenwasserstoffe wie Benzol, Toluol, Xylol oder Erdölfraktionen oder Essigsäure-n-butylester. Bevorzugt sind n-Butanol, Dioxan, Essigsäure-n-butylester, Toluol, Xylol oder Mesitylen.

Die Reaktion kann in einem Temperaturbereich von 0° C - 250° C, bevorzugt bei 100° C - 170° C bei normalen oder erhöhtem Druck durchgeführt werden.

Die Verbindungen der allgemeinen Formel (VII) sind an sich bekannt oder können nach üblicher Methode hergestellt werden [Chem. Pharm. Bull. 30, 1921 (1982), Chem. Pharm. Bull 23, 3106 (1975; J. Org` Chem. 47, 3016 (1982)].

Die Verbindungen der allgemeinen Formel (VIII) sind an sich bekannt oder können nach üblichen Methoden hergestellt werden [J.J. Tufariello in 1,3-Dipolar Cycloaddition Chemistry Vol. 2, Ed. A. Padwa, p-83-168, John Wiley (1984), R. Huisgen, H. Seidel, J. Bruning, Chem. Ber. 102, 1102 (1969)].

Die Verbindungen der allgemeinen Formel (III) sind teilweise bekannt [($R^1$ = Butyl, $R^2$ = Propyl (I)) vgl. PCT, WO 88/02374] und können aber durch das oben angegebene neue Verfahren über die Stufe der Isoxazolidine (Formel II) in der bevorzugten Stereochemie und in besseren Ausbeuten erhalten werden.

Die Verbindungen der allgemeinen Formeln (IV) und (V) sind an sich bekannt und können durch Umsetzung eines entsprechenden Bruchstückes, bestehend aus einer oder mehreren Aminosäureeinheiten, mit einer freien, gegebenenfalls in aktivierter Form vorliegenden Carboxylgruppe mit einem komplementierenden Bruchstück, bestehend aus einer oder mehreren Aminosäureeinheiten, mit einer Aminogruppe, gegebenenfalls in aktivierter Form, und durch Wiederholung dieses Vorgangs mit entsprechenden Bruchstücken hergestellt werden, anschließend können gegebenenfalls Schutzgruppen abspalten oder gegen andere Schutzgruppen austauscht werden [vgl. Houben-Weyl, Methoden der organischen Chemie, Synthese von Peptiden II, 4. Aufl. Bd. 15/1, 15/2, Georg Thieme Verlag, Stuttgart].

Als Hilfsstoffe für die Peptidkupplung und die Einführung des Restes Y (Formeln (IV) und (IVa)) werden

bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N-'Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder Isoxazoliumverbindungen wie 2-Ethyl-5-phenyl-isoxazolium-3-sulfonat oder 2-tert-Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)-phosphonium-hexafluorophosphat.

Als Basen können bei der Peptidkupplung und bei der Umsetzung mit Verbindungen der allgemeinen Formel (VI) Alkalicarbonat z.B. Natrium- oder Kaliumcarbonat oder -hydrogencarbonat, oder organische Basen wie Trialkylamine z.B. Triethylamin, N-Ethylmorpholin, N-Methylpiperidin oder N-Methylmorpholin eingesetzt werden. Bevorzugt ist Triethylamin.

Die Hilfsstoffe und Basen werden in einer Menge von 0,5 Mol bis 4 Mol, bevorzugt 1 bis 1,5 Mol, bezogen auf jeweils 1 Mol der Verbindungen der allgemeinen Formel (VI) eingesetzt.

Die Peptidkupplung wird in einem Temperaturbereich von -20 °C bis 100 °C, vorzugsweise bei 0 bis 25 °C und bei Normaldruck durchgeführt.

Die Einführung der Reste X und Y erfolgt in einem Temperaturbereich von 0 °C bis +90 °C, vorzugsweise bei Raumtemperatur.

Die Reaktionen können sowohl bei Normaldruck als auch bei erhöhtem oder erniedrigtem Druck (beispielsweise 0,5 bis 5 bar), vorzugsweise bei Normaldruck durchgeführt werden.

Die Verbindungen der allgemeinen Formeln (IVa) und (VI) sind an sich bekannt oder können nach üblicher Methode hergestellt werden.

Die Abspaltung der jeweiligen Schutzgruppen vor den einzelnen Peptidknüpfungen erfolgt in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen z.B. Methanol, Ethanol oder Isopropanol [vgl. Protective Groups in Organic Synthesis, W. Greene, John Wiley & Sons, New York, 1981; Chemistry and Biochemistry of the Amino Acids, G.C. Barrett, Chapman and Hall, London, New York, 1985].

## In vitro Test

Die inhibitorische Stärke der erfindungsgemäßen Peptide gegen endogenes Renin vom Humanplasma wird in vitro bestimmt. Gepooltes Humanplasma wird unter Zusatz von Ethylendiamintetraessigsäure (EDTA) als Antikoagulanz erhalten und bei -20 °C gelagert. Die Plasmareninaktivität (PRA) wird als Bildungsrate von Angiotensin I aus endogenem Angiotensinogen und Renin nach Inkubation bei 37 °C bestimmt. Die Reaktionslösung enthält 150 µl Plasma, 3 µl 6,6%ige 8-Hydroxychinolinsulfatlösung, 3 µl 10%ige Dimercaprollösung und 144 µl Natriumphosphatpuffer (0,2 M; 0,1% EDTA; pH 5,6) mit oder ohne den erfindungsgemäßen Stoffen in verschiedenen Konzentrationen. Das pro Zeiteinheit gebildete Angiotensin I wird mit einem Radioimmunoassay (Sorin Biomedica, Italien) bestimmt. Die prozentuale Inhibition der Plasmareninaktivität wird berechnet durch Vergleich der hier beanspruchten Substanzen. Der Konzentrationsbereich, in dem die hier beanspruchten Substanzen eine 50% Inhibition der Plasmareninaktivität zeigen, liegt zwischen $10^{-4}$ und $10^{-9}$ M.

Die eingesetzte Standardkonzentration der folgenden Renininhibitoren beträgt 50 µg/ml. Bei mehr als 90% Hemmung wurden die $IC_{50}$-Werte bestimmt.

| Beispiel-Nr. | in vitro 50 µg/ml [%] | $IC_{50}$ [M] |
|---|---|---|
| 1 | 100% | $2{,}0 \times 10^{-9}$ |
| 2 | 100% | $1{,}3 \times 10^{-9}$ |
| 7 | 100% | $7{,}4 \times 10^{-7}$ |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der

Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Anlage I

1. Aminosäuren

Im allgemeinen erfolgt die Bezeichnung der Konfiguration durch das Vorausstellen eines L bzw. D vor der Aminosäureabkürzung, im Fall des Racemats durch ein D,L-, wobei zur Vereinfachung bei L-Aminosäuren die Konfigurationsbezeichnung unterbleiben kann und dann nur im Fall der D-Form bzw. des D,L-Gemisches explizierte Bezeichnung erfolgt.

a) natürliche Aminosäuren

| | |
|---|---|
| Ala | L-Alanin |
| Asp | L-Asparaginsäure |
| Cys | L-Cystein |
| Gln | L-Glutamin |
| Glu | L-Glutaminsäure |
| Gly | L-Glycin |
| His | L-Histidin |
| Ile | L-Isoleucin |
| Leu | L-Leucin |
| Lys | L-Lysin |
| Phe | Phenylalanin |

| | |
|---|---|
| Cpg | Cyclopentylglycin |
| Val | Valin |

2. Aktivierungsreagenzien und Additive

| | |
|---|---|
| HOBT | 1-Hydroxybenzotriazol |
| HOSU | N-Hydroxysuccinimid |
| DCC | Dicyclohexylcarbodiimid |
| NMM | N-Methylmorpholin |
| BOP | Benzotriazolyloxy-tris-(dimethylamino)-phosphoniumhexafluorophosphat |

3. Schutzgruppen

| | |
|---|---|
| BOC | Tert-Butoxycarbonyl |
| Z | Benzyloxycarbonyl |
| AMP | 2-Aminomethylpyridyl |
| BOM | Benzyloxymethyl |
| PAA | Pyridylacetyl |

Anlage II

Die folgenden Laufmittelsysteme wurden verwandt:

| | | |
|---|---|---|
| A | Ether:Hexan | 2:8 |
| B | Ether:Hexan | 3:7 |
| C | Ether:Hexan | 4:6 |
| D | Ether:Hexan | 7:3 |
| E | $CH_2Cl_2$:$CH_3OH$ | 95:5 |
| F | $CH_2Cl_2$:$CH_3OH$ | 98:2 |
| G | $CH_2Cl_2$:$CH_3OH$ | 90:10 |
| H | $CH_2Cl_2$:$CH_3OH$:$NH_3$ | 95:5:0,1 |
| I | Toluol:Essigester | 1:1 |

| | | |
|---|---|---|
| J | $CH_2Cl_2$:$CH_3OH$:$NH_3$ | 90:90:0,1 |
| K | Tol:Essigsäureethylester:$CH_3OH$ | 25:75:1 |
| L | nBuOH:HOAc:$H_2O$ | 8:2:2 |
| M | Tol:Essigsäureethylester | 1:1 |
| N | Tol:Essigsäureethylester | 1:3 |

Für die Verbindungen, die mit x gekennzeichnet sind, liegen $^1$H-NMR-Daten vor. Der in den Beispielen aufgeführte Rest † steht für die tert.-Butylgruppe.

Ausgangsverbindungen

Beispiel I

BOC-Phenylalanin

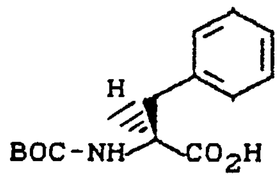

300 g (1,91 mol) L-Phenylalanin werden in 360 ml Dioxan und 360 ml $H_2O$ suspendiert. 432,9 g (1,98 mol) Di-tert.-butyldicarbonat werden unter Rühren bei pH 9,8 zugegeben. Der pH wird mit ca. 975 ml 4n NaOH konstant gehalten. Nach 16h wird das Reaktionsgemisch mit Ether extrahiert, die wäßrige Phase wird mit Zitronensäure auf pH 3-4 eingestellt und anschließend mit 2 x Ether, 2 x Essigester extrahiert. Die organischen Phasen werden vereinigt und 3 x mit Wasser gewaschen. Nach Einrotieren und Kristallisation aus Diethylether/Hexan erhält man 291,6 g (60,7 %).
Fp: 88-89° C
NMR (DMSO, 300 MHz): δ = 1,35 (s; 9H, $C(CH_3)_3$).

Beispiel II

BOC-Cyclohexylalanin

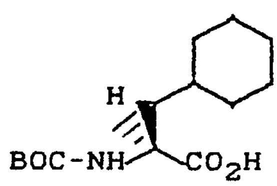

265 g (1,0 mol) der Verbindung aus Beispiel I werden in 2 l Methanol gelöst und über 20 g 5 % Rh/C 5h bei 40 atm hydriert. Der Katalysator wird über Celite abgesaugt, mit Methanol gewaschen und die erhaltene Lösung eingeengt. Es werden 271 g (100 %) des Beispiels 6 erhalten.
NMR (DMSO, 300 MHz): δ = 0,8-1,8 (m; 22H, Cyclohexylmethylen, $C(CH_3)_3$.

Beispiel III

BOC-Cyclohexylalanin-N-methyl-N-methoxyamid

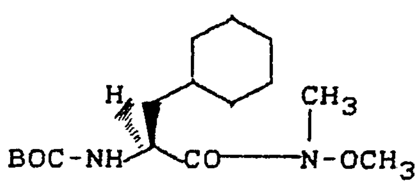

163,0 g (0,601 mol) der Verbindung aus Beispiel II und 40,3 g (0,661 mol) N,O-Dimethylhydroxylamin werden in 2 l Methylenchlorid bei Raumtemperatur gelöst. Bei 0° C werden 303,5 g (3,005 mol) Triethylamin zugetropft (pH - 8). Bei max. -10° C wird 390,65 ml einer 50 %igen Lösung (0,601 mol) von n-Propylphosphonsäureanhydrid in Methylenchlorid zugetropft. Über Nacht wird auf 25° C erwärmt und 16h gerührt. Anschließend wird die Reaktionslösung eingeengt, der Rückstand mit 500 ml ges. Bicarbonatlösung versetzt und 20 min, bei 25° C gerührt. Nach dreimaliger Extraktion mit Essigester wird die organische Phase über $Na_2SO_4$ getrocknet und eingeengt. Rohausbeute: 178 g (94,6 %)˙ Das Rohmaterial wird an Kieselgel chromatographiert (Laufmittel F).
Ausbeute: 136,6 9 (72,3 %).

NMR (DMSO, 300 MHz): δ = 1,37 (s; 9H, $C(CH_3)_3$), 3,08 (s; 3H, $N-CH_3$); 3,71 (s; 3H; $O-CH_3$).

Beispiel IV

BOC-Cyclohexylalaninal

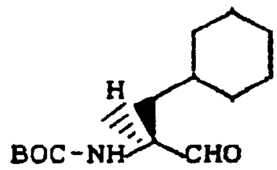

In einer ausgeheizten Apparatur werden unter Stickstoff 63,7 g (0,21 mol) der Verbindung aus Beispiel III in 1,5 l aloxiertem Ether gelöst, bei 0° C werden 10 g (0,263 mol) $LiAlH_4$ portionsweise dazugegeben, und anschließend 20 min. bei 0° C gerührt. Danach wird vorsichtig eine Lösung von 50 g (0,367 mol) $KHSO_4$ in 1 l $H_2O$ bei 0° C dazugetropft. Die Phasen werden getrennt, die wäßrige Phase noch 3 x mit Diethylether 300 ml extrahiert, die vereinigten organischen Phasen dreimal mit 3n HCl, 3 x mit $NaHCO_3$-Lösung und 2 x mit NaCl-Lösung gewaschen. Die organische Phase wird über $Na_2SO_4$ getrocknet und eingeengt. Ausbeute: 45 g (84,1 %). Der Aldehyd wird entweder sofort weiterverarbeitet oder ein bis zwei Tage bei -24° C gelagert.
NMR (DMSO, 300 MHz): δ = 9,41 (s; 1H, -CHO).

Beispiel V

BOC-Allylamin

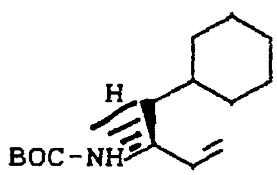

14,6 9 (35 mmol) "Instant Ylide" (Fluka 69500) werden in 90 ml wasserfreiem Tetrahydrofuran suspendiert. Unter Eiskühlung wird bei einer Reaktionstemperatur zwischen 20 und 25° C eine Lösung von 9,0 g (35 mmol) BOC-Cyclohexylalaninal in 45 ml wasserfreiem Tetrahydrofuran zugetropft. Nach 15 min. Rühren wird das Reaktionsgemisch auf 250 ml Eis gegossen und zweimal mit je 150 ml Essigester/n-Hexan 3:1 extrahiert. Nach Trocknen über $Na_2SO_4$ und Einengen wird der Rückstand an Kieselgel chromatographiert (Laufmittel D).
Ausbeute: 3,2 g (40,0 %)
EI-MS: m/z = 253 (0,1 % M+H), 197 (9 %).

Beispiel VI

BOC-Allylamin

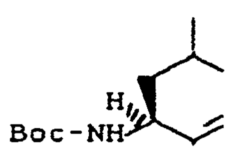

Darstellung erfolgt analog der Vorschrift aus Beispiel V mit einem 0,24 Mol-Ansatz.

Ausbeute: 25,92 g (50,6 %).

Beispiel VII

2-Benzyl-3(1-methylethyl)-5-[1-tert.butoxycarbonylamino-2-cyclohexylethyl]-isoxazolidin

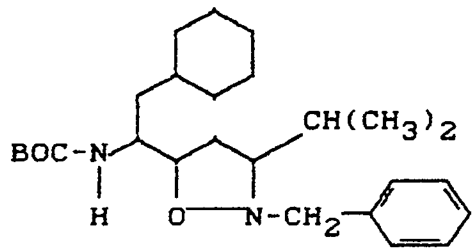

202,4 9 (0,8 mol) der Verbindung aus Beispiel V werden in 1000 ml Mesitylen gelöst und auf 140 °C am Wasserabscheider erwärmt. Bei dieser Temperatur wird eine Mischung von 197 g (1,6 mol) N-Benzylhydroxylamin und 137,68g (1,6 mol) Isobutyraldehyd in 800 ml Mesitylen über 2h zugetropft. Nach 4h und 8h Reaktionszeit wird die gleiche Menge N-Benzylhydroxylamin, Isobutyraldehyd in Mesitylen zugetropft. Nach insgesamt 16h Reaktionszeit wird der Ansatz eingeengt, der Rückstand mit Diethylether versetzt und anschließend mit 1m $KHSO_4$-Lösung gewaschen. Nach Trocknen über $Na_2SO_4$ und Einengen wurde an Kieselgel chromatographiert (Laufmittel B).
Ausbeute: 168 g (52,2 % der Theorie)
Es wurden 4 Diastereomere erhalten:

| Diastereomer | Ausbeute | DC, $R_f$ (B) | $^1$H-NMR C-4-NH |
|---|---|---|---|
| a) 1S 3S 4S | 11 g | 0,42 | 6,37 |
| b) 1R 3R 4S | 10 g | 0,29 | 6,57 |
| c) 1R 3S 4S | 69 g | 0,25 | 6,41 |
| d) 1S 3R 4S | 34 g | 0,18 | 6,63 |

Die in Tabelle I aufgeführten Beispiele wurden in Analogie zur Vorschrift des Beispiels VII hergestellt:

Tabelle I

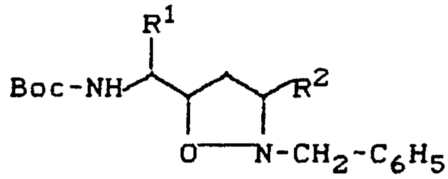

| Bsp.-Nr. | $R^1$ | $R^2$ | FAB-MS M+H[%] |
|---|---|---|---|
| VIII | $-CH_2-CH(CH_3)_2$ | $-CH(CH_3)_2$ | 391 (55) Isomer B |
| IX | $-CH_2-C_6H_{11}$ | $-CH_3$ | 403 (51) |

Beispiel X

(2R, 4S, 5S)-2-Amino-5-(tert.butoxycarbonylamino)-6-cyclohexyl-4-hydroxyhexan

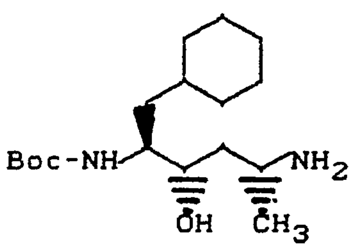

18,1 g (45 mmol) der Verbindung aus Beispiel IX (Diastereomer C) werden in 300 ml Methanol gelöst. Nach Zugabe von 14,2 9 (225 mmol) Ammoniumformiat wird intensiv mit $N_2$ gespült und 3,6 g Palladium/Kohle (10%) zugegeben. Unter Rückfluß wird 3 h gerührt. Nach Abkühlen wird der Katalysator abfiltriert, die Lösung eingeengt, in Essigsäureethylester gelöst und zweimal mit gesättigter Bicarbonatlösung gewaschen. Die organische Phase wird über Natriumsulfat getrocknet, filtriert, eingeengt und im Hochvakuum getrocknet.

Ausbeute: 11,36 g (80,3% der Theorie)

$R_f$ = 0,27 (I)

Die in Tabelle II aufgeführten Verbindungen wurden in Analogie zur Vorschrift des Beispiels X hergestellt.

Tabelle II

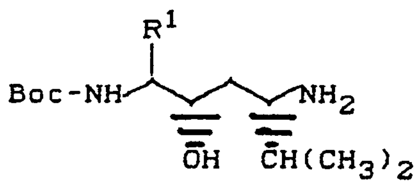

| Bsp.-Nr. | $R^1$ | FAB-MS M+H [%] |
|---|---|---|
| XI | $-CH_2-CH(CH_3)_2$ | 303 ( 95) |
| XII | $-CH_2-C_6H_{11}$ | 343 (100) |

Beispiel XIII

(2R, 4S, 5S)-2-(Valerylamino)-5-(tert.butoxycarbonylamino)-6-cyclohexyl-4-hydroxyhexan

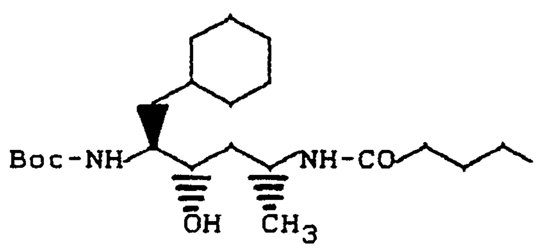

6,6 g (21 mmol) der Verbindung aus Beispiel X werden in 500 ml Methylenchlorid gelöst. Unter Feuchtigkeitsausschluß ($CaCl_2$-Rohr) wird eine Lösung von Pentansäureanhydrid [bereitet aus 2,16 g (21 mmol) Pentansäure und 2,16 g (10,5 mmol) Dicyclohexylcarbodiimid in 50 ml Methylenchlorid, Filtration] in Methylenchlorid bei Raumtemperatur zugegeben. Nach 3 h wird eingeengt, in Essigsäureethylester aufgenommen, mit gesättigter Bicarbonatlösung gewaschen und über Natriumsulfat getrocknet. Nach Filtration und Einengen wird im Hochvakuum getrocknet.

Ausbeute: 8,0 g (95,2% der Theorie)

$R_f$ = 0,74 (G)

FAB-MS: m/z = 421 (12%, M + Na)

Die in Tabelle III aufgeführten Verbindungen wurden in Analogie zur Vorschrift des Beispiels XIII hergestellt.

Tabelle III

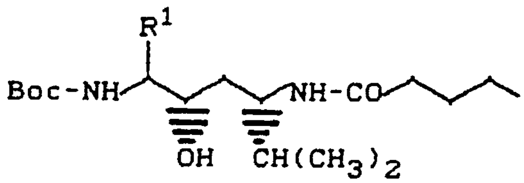

| Bsp.-Nr. | $R^1$ | FAB-MS M+H [%] |
|---|---|---|
| XIV | $-CH_2-CH(CH_3)_2$ | 387 (100) |
| XV | $-CH_2-C_6H_{11}$ | 327 (3, M+H-Boc) |

Beispiel XVI

(2R, 4S, 5S)-2-(Valerylamino)-5-[Nα-(tert.butoxycarbonyl)-Nπ -(tert.butoxycarbonyl)-L-histidylamino]-6-cyclohexyl-4-hydroxyhexan

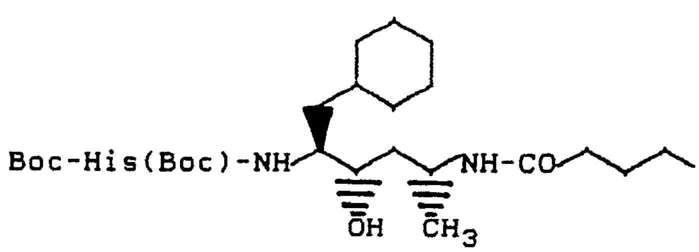

7,57 g (19 mmol) der Verbindung aus Beispiel XIII werden in 70 ml 4 n Salzsäure/Dioxan 30 min unter Feuchtigkeitsausschluß gerührt. Die Lösung wird eingeengt, mit Diethylether versetzt und zur Trockne eingedampft. Nach Trocknen im Hochvakuum werden 5,54 g (16,5 mmol) des entsprechenden Hydrochlorids, 4,46 g (33 mmol) HOBT und 5,86 g (16,5 mmol) Boc-His(Boc)OH in 500 ml Methylenchlorid gelöst. Nach Kühlen auf 0°C wird mit N-Methylmorpholin auf pH 8,5 eingestellt und 3,57 g (17,3 mmol) Dicyclohexylcarbodiimid zugegeben. Nach 16 h bei 20°C wird der Harnstoff abfiltriert, die Lösung eingeengt, in Essigsäureethylester aufgenommen und mit gesättigter Bicarbonatlösung gewaschen. Nach Trocknen über Natriumsulfat wird eingeengt und im Hochvakuum getrocknet.

Ausbeute: 8,33 g (79,5% der Theorie)

$R_f$ = 0,61 (G)

FAB-MS: m/z = 636 (79%, M+H)

Die in Tabelle IV aufgeführten Beispiele wurden in Analogie zur Vorschrift des Beispiels XVI hergestellt.

Tabelle IV

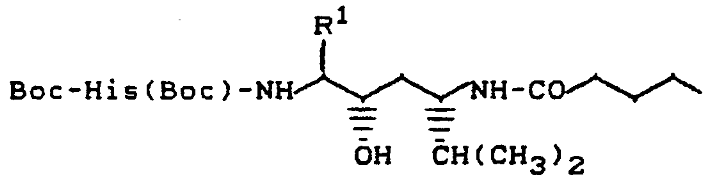

| Bsp.-Nr. | $R^1$ | FAB-MS M+H [%] |
|---|---|---|
| XVII | $-CH_2-CH(CH_3)_2$ | 624 (16) |
| XVIII | $-CH_2-C_6H_{11}$ | 670 (44, M+Li) |

Beispiel XIX

(2R, 4S, 5S)-2-(Valerylamino)-5-[Nα-(tert.butoxycarbonyl)-L-cyclopentylglycylamino]-6-cyclohexyl-4-hydroxyhexan

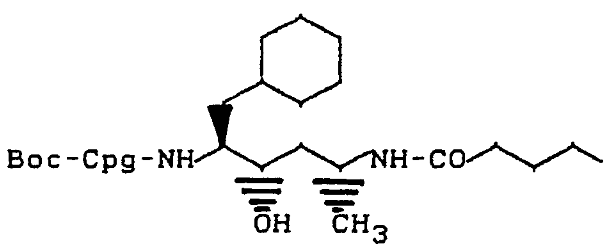

5,6 g (24,4 mmol) BocCpgOH werden in 50 ml wasserfreiem Tetrahydrofuran gelöst. Nach Zugabe von 2,7 ml (24,4 mmol) N-Methylmorpholin wird bei -20°C 3,2 ml (24,4 mmol) Chlorameisensäureisobutylester zugetropft und 15 min. bei -20°C gerührt. Zu dieser Lösung werden 5,45 g (16,3 mmol) der deblockierten Verbindung aus Beispiel XIII und 1,8 ml (16,3 mmol) der N-Methylmorpholin in 50 ml Tetrahydrofuran/Wasser 1:1 zugetropft und innerhalb von 30 min auf
20°C erwärmt. Nach weiteren 30 min wird die Reaktionslösung eingeengt, in 1 l Diethylether gegeben und auf 0°C gekühlt. Nach 16 h werden 4,6 g (54,0%) Kristalle abgesaugt. Die Mutterlauge wird mit gesättigter Bicarbonatlösung gewaschen, die Etherphase über Natriumsulfat getrocknet, eingeengt und im Hochvakuum getrocknet. Es werden 6 g gelbes Öl erhalten, das an Kieselgel chromatographiert (E) wird.
Ausbeute: 3,31 g (38,8% der Theorie)
$R_f$ = 0,79 (G)

FAB-MS: m/z = 524 (38%, M+H)

Herstellungsbeispiele

Beispiel 1

(2R, 4S, 5S)-2-(Valerylamino)-5-[Nα-(2-S-benzyl-3-tert.-butylsulfonylpropionyl)-L-histidylamino]-6-cyclohexyl-4-hydroxyhexan

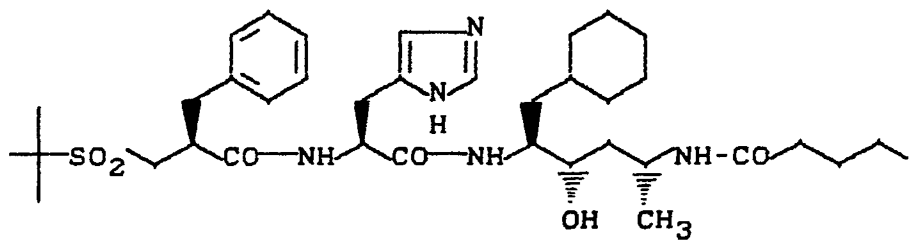

1,145 g (1,8 mmol) der Verbindung aus Beispiel XVI werden in 11 ml 4n Salzsäure/Dioxan 30 min gerührt. Die Lösung wird eingeengt, mit Diethylether versetzt und zur Trockene eingedampft. Nach Trocknen im Hochvakuum werden 875,5 mg (1,8 mmol) des entstandenen Dihydrochlorids in 50 ml Methylenchlorid gelöst und auf 0°C gekühlt. Nach Zugabe von 509,4 mg (1,8 mmol) der β-tert.Butylsulfonyl-α -benzylpropionsäure wird mit Triethylamin auf pH 8 eingestellt und mit 875,2 mg (1,98 mmol) BOP versetzt. Nach 16 h Reaktion bei Raumtemperatur wird eingeengt, in Essigsäureethylester aufgenommen und 3 mal mit gesättigter Bicarbonatlösung gewaschen. Es werden 494,8 mg (39,2% der Theorie) erhalten, die an Kieselgel chromatographiert werden (G).

$R_f$ = 0,24 (G)

FAB-MS: m/z = 702 (100%, M+H)

Beispiel 2

(2R, 4S, 5S)-2-(Valerylamino)-5-{Nα-[4-(morpholinocarbonyl)-L-phenylalanyl]-L-cyclopentylglycylamino} -6-cyclohexyl-4-hydroxyhexan

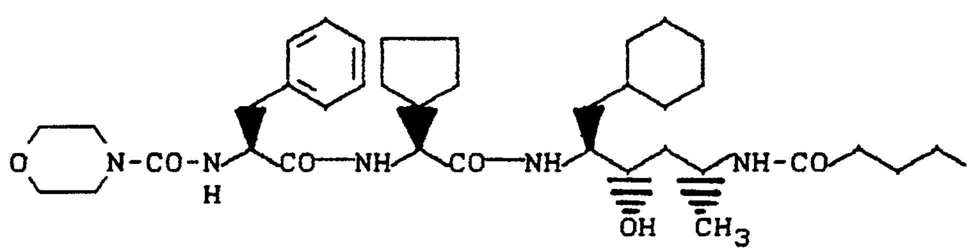

1,044 g (3,75 mmol) Morpholinocarbonylphenylalanin werden in 50 ml wasserfreiem Tetrahydrofuran gelöst. Nach Zugabe von 0,4 ml (3,75 mmol) N-Methylmorpholin wird bei -20°C 0,48 ml (3,75 mmol) Chlorameisensäureisobutylester zugetropft und 15 min bei -20°C gerührt. In dieser Lösung wird 1,17 g (2,5 mmol) der deblockierten Verbindung aus Beispiel XIX in 50 ml Tetrahydrofuran/Wasser 1:1 [mit N-Methylmorpholin auf pH 8 eingestellt] getropft und während 16 h auf 20°C erwärmt. Die Aufarbeitung erfolgt wie in Beispiel XIX beschrieben.

Kristalle: 944,5 mg (55,5% der Theorie)

Öl 800 mg: nach Säulenchromatographie 108,2 mg (6,4% der Theorie)

Gesamtausbeute: 1,053 g (64,9% der Theorie)

$R_f$ = 0,62 (G)

FAB-MS: m/z = 624 (100% M+H)

Die in Tabelle 1 aufgeführten Beispiele wurden in Analogie zur Vorschrift des Beispiels 1 hergestellt:

Tabelle 1

| Bsp.-Nr. | Formel |
|---|---|
| 3 | 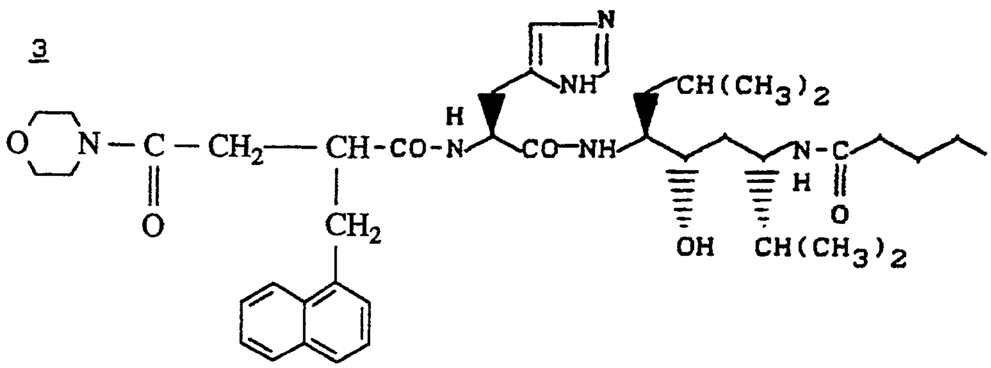 |
| NMR: X<br>FAB-MS: 733 (100%, M+H); | Summenformel: $C_{41}H_{60}N_6O_6$<br>$R_f$ = 0,41 (I) |

Fortsetzung Tabelle 1

Bsp.-Nr. Formel

4

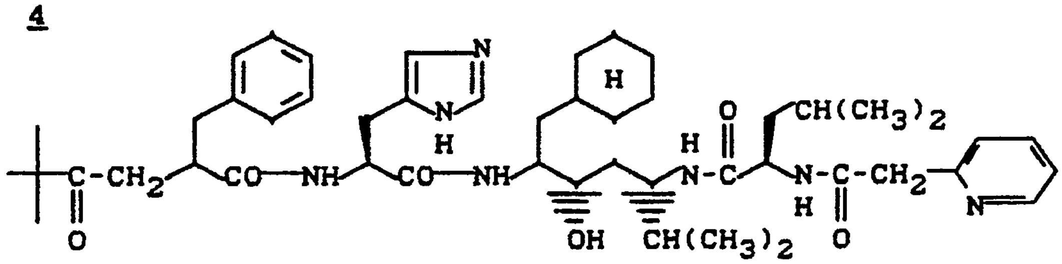

NMR: X Summenformel: $C_{48}H_{71}N_7O_6$
FAB-MS: 842 (61%, M+H); $R_f$: 0,22 (G)

5

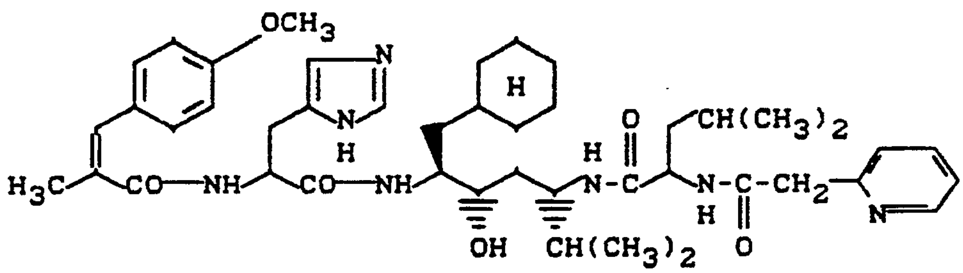

NMR: X Summenformel: $C_{44}H_{63}N_7O_6$
FAB-MS: 786 (53%, M+H); $R_f$: 0,38 (G)

6

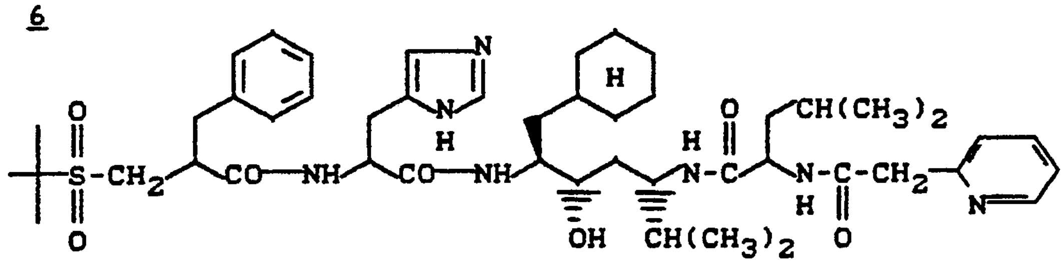

NMR: X Summenformel: $C_{47}H_{71}N_7O_7S$
FAB-MS: 878 (100% M+H); $R_f$: 0,37 (I) Isomer (a)

Fortsetzung Tabelle 1

| Bsp.-Nr. | Formel |
|---|---|
| 7 | 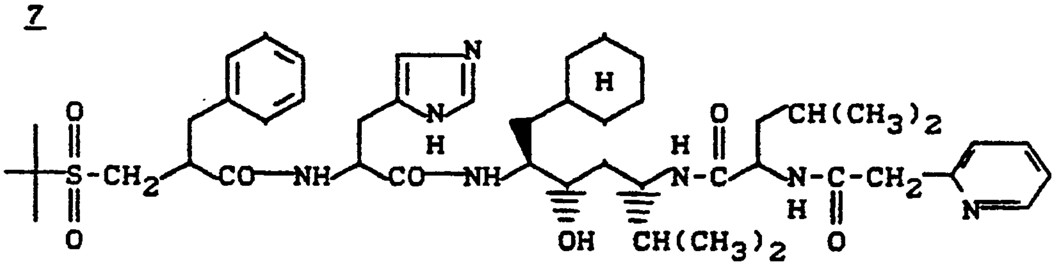 |
| | NMR: X<br>FAB-MS: 878 (50%, M+H); Summenformel: $C_{47}H_{71}N_7O_7S$<br>$R_f$: 0,37 (I) Isomer (b) |
| 8 | 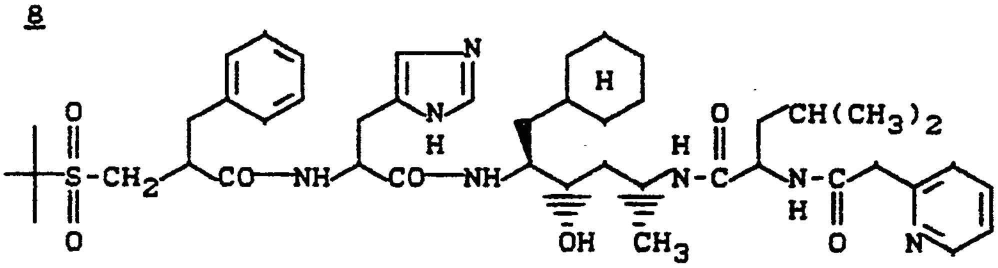 |
| | NMR: X<br>FAB-MS: 850 (100% M+H); Summenformel: $C_{45}H_{67}N_7O_7S$<br>$R_f$: 0,42 (I) |
| 9 | 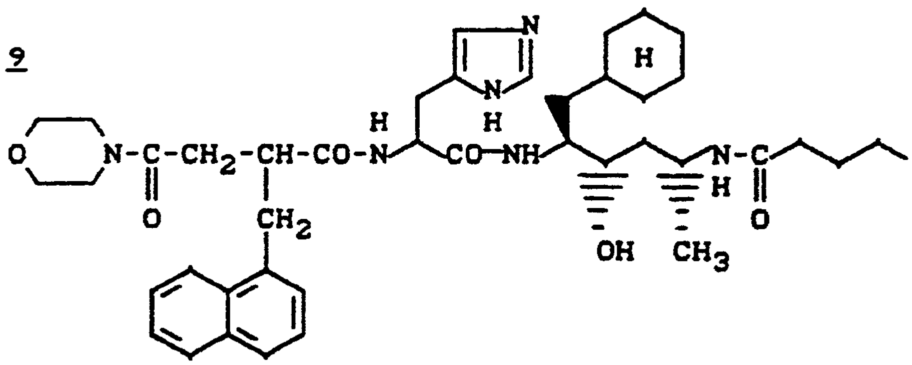 |
| | NMR: X<br>FAB-MS: 745 (61%, M+H); Summenformel: $C_{42}H_{60}N_6O_6$<br>$R_f$ = 0,32 (I) |

Fortsetzung Tabelle 1

| Bsp.-Nr. | Formel |
|---|---|

10

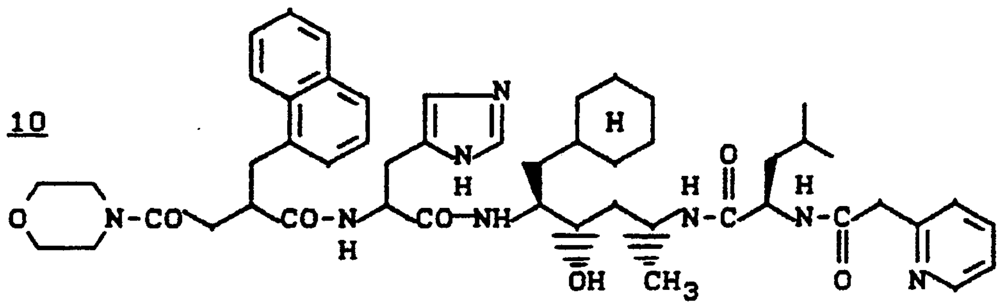

NMR: X  Summenformel: $C_{50}H_{68}N_8O_7$
FAB-MS: 893 (30%, M+H);  $R_f$ = 0,48 (I)

---

11

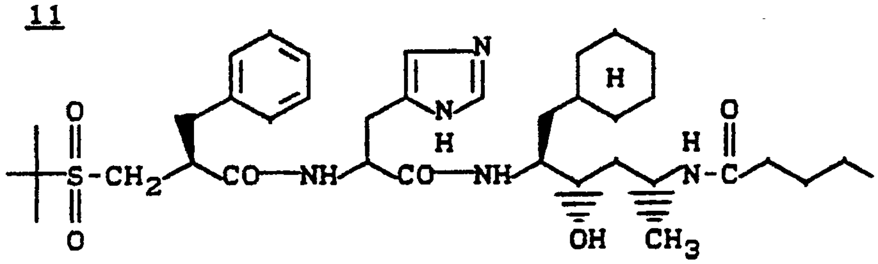

NMR: X  Summenformel: $C_{37}H_{59}N_5O_6S$
FAB-MS: 702 (100% M+H);  $R_f$: 0,24 (G)

---

12

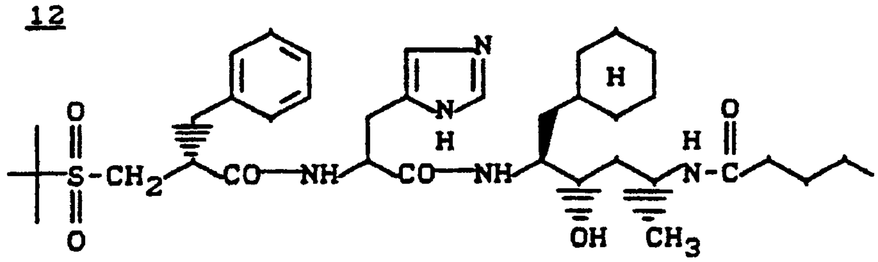

NMR: X  Summenformel: $C_{37}H_{59}N_5O_6S$
FAB-MS: 702 (31%, M+H);  $R_f$: 0,38 (G)

---

Die in Tabelle 2 aufgeführten Verbindungen wurden in Analogie zur Vorschrift des Beispiels 2 hergestellt:

Tabelle 2

| Bsp.-Nr. | Formel |
|---|---|
| 13 | 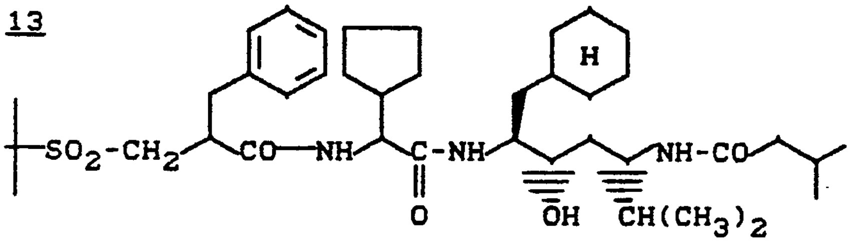 |
| | NMR: X<br>FAB-MS: 718 (90% M+H); Summenformel: $C_{40}H_{67}N_3O_6S$ |
| 14 | 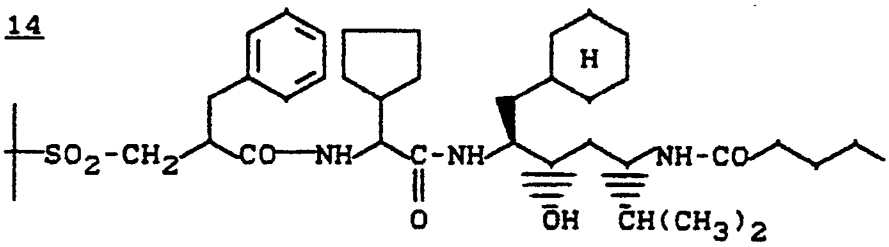 |
| | NMR: X<br>FAB-MS: 718 (100% M+H); Summenformel: $C_{40}H_{67}N_3O_6S$ |
| 15 | 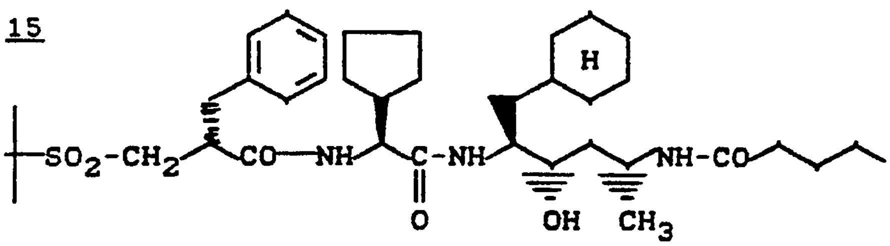 |
| | NMR: X<br>FAB-MS: 690 (75% M+H); Summenformel: $C_{38}H_{63}N_3O_6S$<br>$R_f$ = 0,33 (M) |

Fortsetzung Tabelle 2

| Bsp.-Nr. | Formel |
|---|---|

16

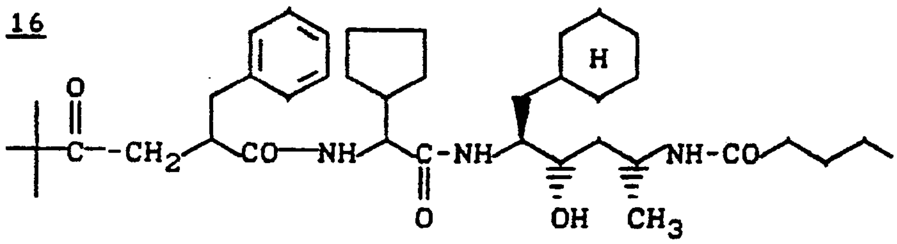

NMR: X Summenformel: $C_{39}H_{63}N_3O_5$
FAB-MS: 654 (100% M+H); $R_f$ = 0,60 (G)

17

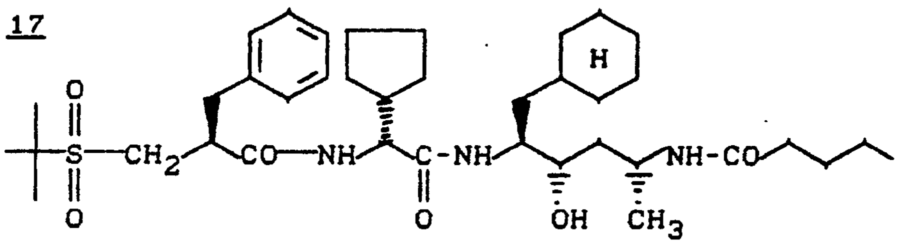

NMR: X Summenformel: $C_{38}H_{63}N_3O_6S$
FAB-MS: 690 (100% M+H); $R_f$ = 0,42 (M)

18

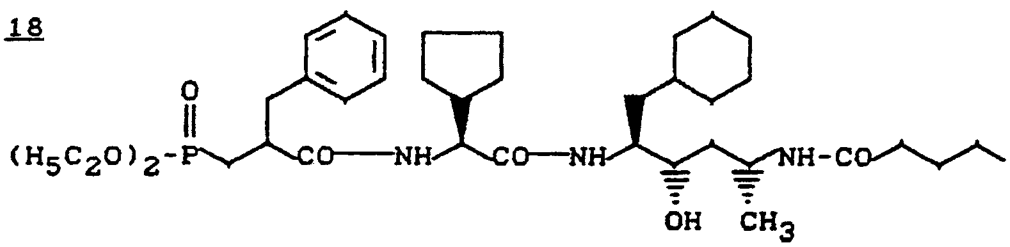

NMR: X Summenformel: $C_{38}H_{64}N_3O_6P$
FAB-MS: 705 (100 %) $R_f$ = 0,58 (G)

Fortsetzung Tabelle 2

| Bsp.-Nr. | Formel |
|---|---|

19

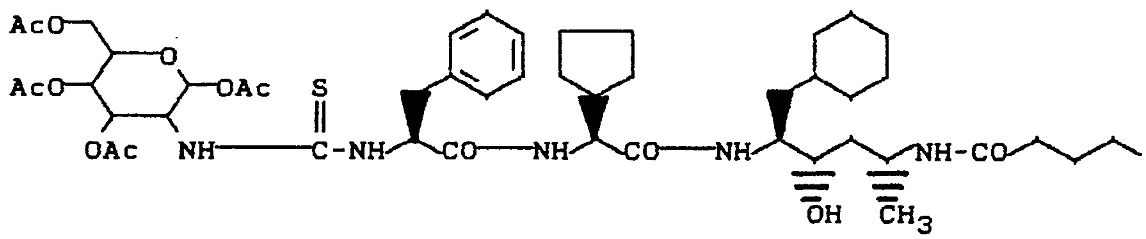

NMR: X
FAB-MS: 966 (10 %, M+Li)

Summenformel: $C_{48}H_{73}N_5O_{13}S$
$R_f$ = 0,69 (G)

20

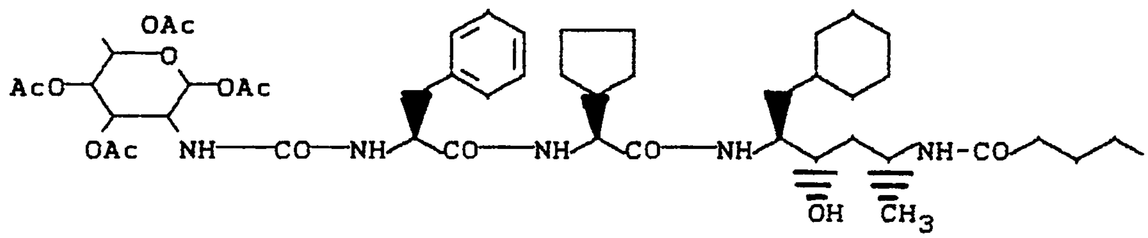

NMR: X
FAB-MS: 950 (40 %, M+Li)

Summenformel: $C_{48}H_{73}N_5O_{14}$
$R_f$ = 0,64 (G)

21

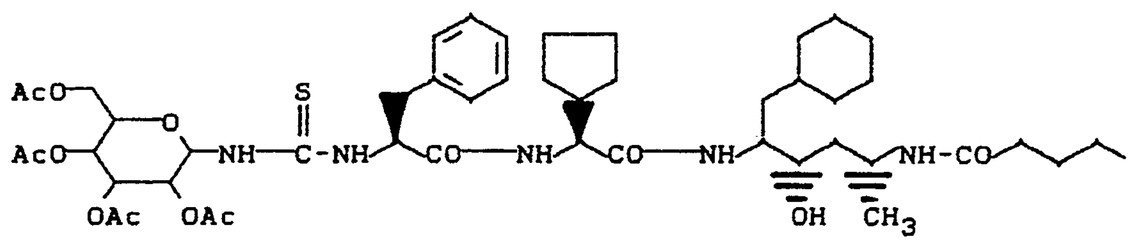

NMR: X
FAB-MS: 966 (100 %, M+Li)

Summenformel: $C_{48}H_{73}N_5O_{13}S$
$R_f$ = 0,44 (G)

Fortsetzung Tabelle 2

| Bsp.-Nr. | Formel |
|---|---|

22

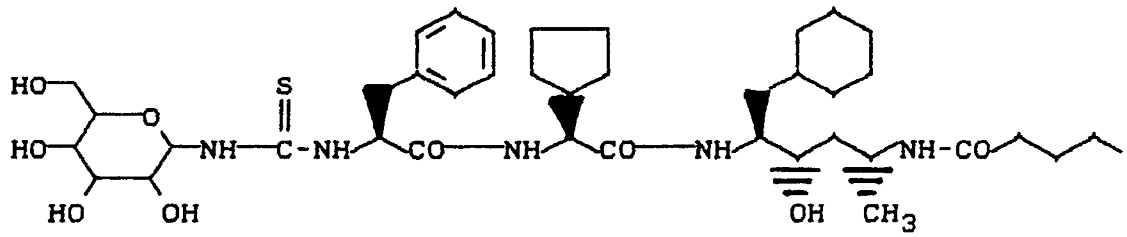

NMR: X
Summenformel: $C_{40}H_{65}N_5O_9S$

FAB-MS: 798 (M+Li)
$R_f$ = 0,53 (G)

23

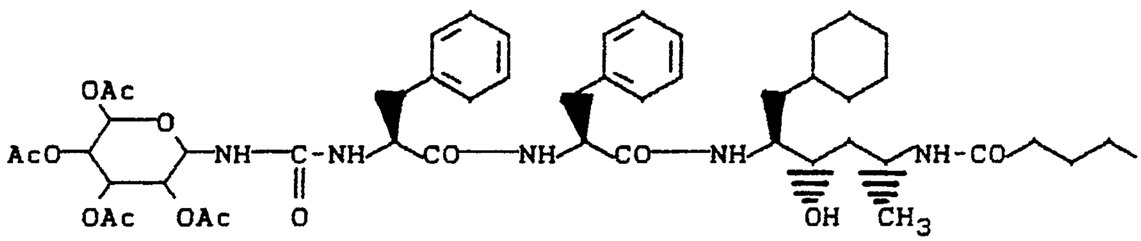

NMR: X
Summenformel: $C_{50}H_{71}N_5O_{14}$

FAB-MS: 986 (18 %, M+H)
$R_f$ = 0,45 (G)

24

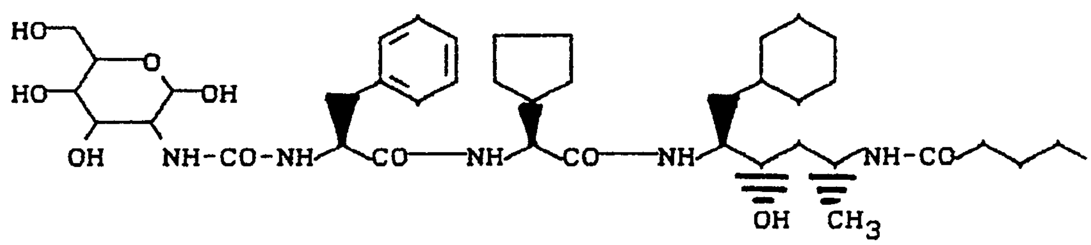

NMR: X
Summenformel: $C_{40}H_{61}N_5O_8$

FAB-MS: 746 (100 %, M+Li)
$R_f$ = 0,40 (G)

Fortsetzung Tabelle 2

Bsp.-Nr. | Formel

25

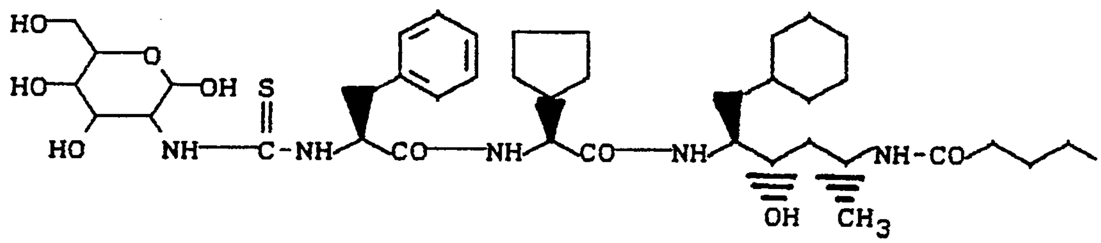

NMR: X
FAB-MS: 780 (100 %, M+Li)

Summenformel: $C_{40}H_{63}N_5O_8S$
HPLC

26

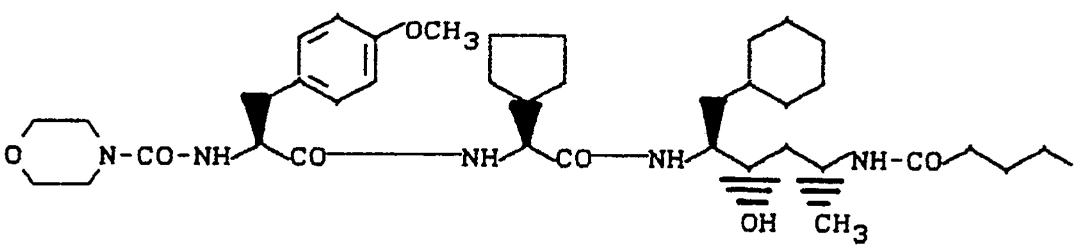

NMR: X
FAB-MS: 714 (75 % M+H)

Summenformel: $C_{39}H_{63}N_5O_7$
$R_f$ = 0,39 (G)

27

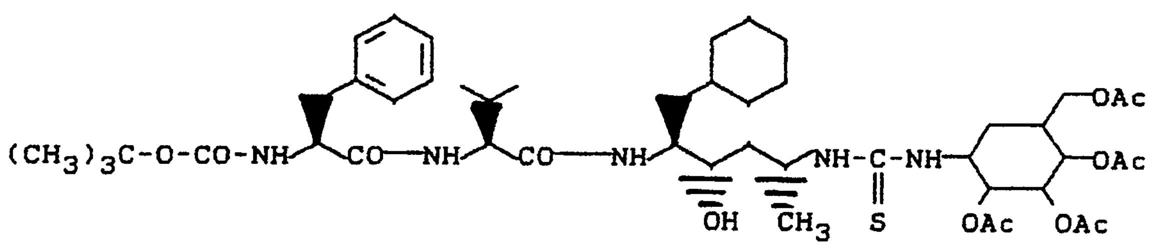

NMR: X
FAB-MS: 950 (10 %, M+H)

Summenformel: $C_{46}H_{71}N_5O_{14}S$
$R_f$ = 0,43 (G)

Fortsetzung Tabelle 2

| Bsp.-Nr. | Formel |
| --- | --- |

28

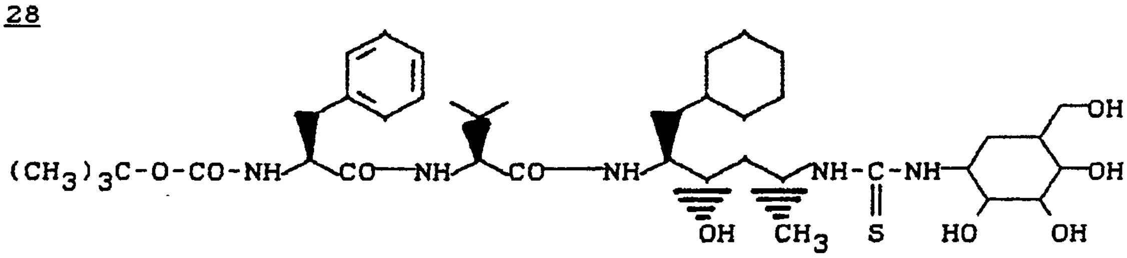

NMR: X

FAB-MS: 782 (87 %, M+H))

Summenformel: $C_{38}H_{63}N_5O_{10}S$

$R_f$: 0,19 (G)

29

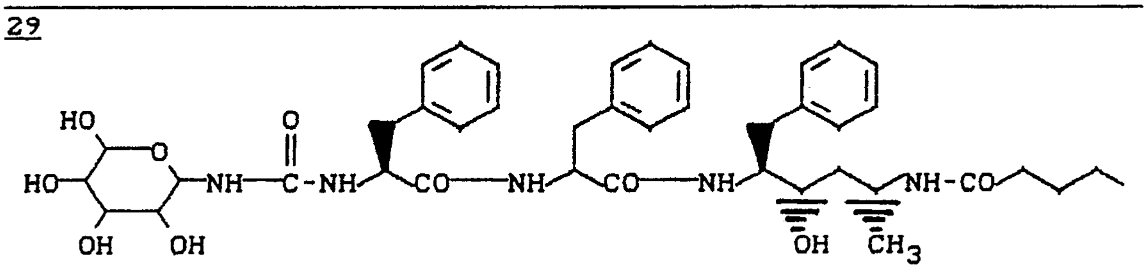

NMR: X

FAB-MS: 784 (95 %, M+H)

Summenformel: $C_{41}H_{61}N_5O_{10}$

$R_f$ = 0,12 (G)

30

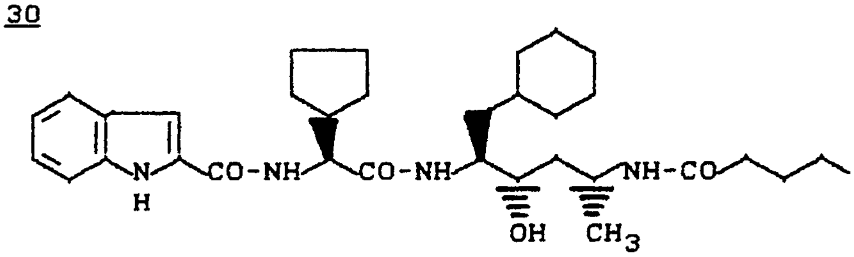

NMR: X

FAB-MS: 573 (23 %, M+Li)

Summenformel: $C_{23}H_{50}N_4O_4$

$R_f$ = 0,35 (I)

31

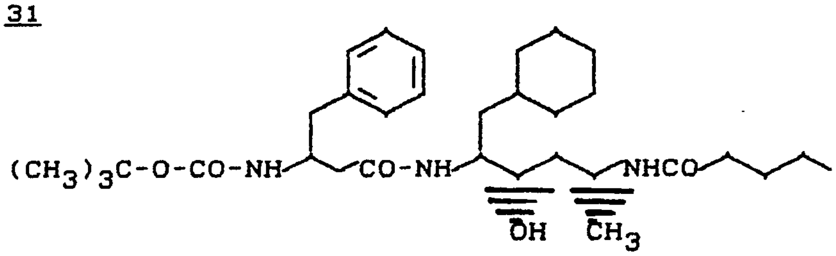

NMR: X

FAB-MS: 566 (100 %, M+Li)

Summenformel: $C_{32}H_{53}N_3O_5$

$R_f$ = 0,51 (G)

## Patentansprüche

**1.** Retroisostere acylierte Dipeptide der allgemeinen Formel (I)

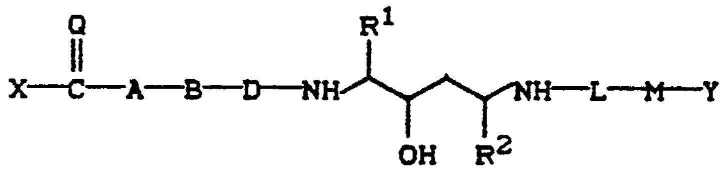 (I)

in welcher
X - für Indolyl, tert.-Butoxy, Morpholino oder für eine Gruppe der Formel

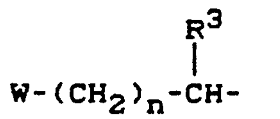 ,

$R^4$-NH- ,

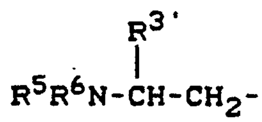

oder für T steht,

worin
$R^3$ und $R^{3'}$ gleich oder verschieden sind und geradkettiges oder verzweigtes Alkyl mit bis zu 8 Kohlenstoffatomen bedeuten, das gegebenenfalls durch Aryl mit 6 bis 10 Kohlenstoffatomen substituiert ist,
n - eine Zahl 1, 2, 3 oder 4 bedeutet,
W - eine Gruppe der Formel
$R^7$-CO-, $R^8$-$SO_2$- oder

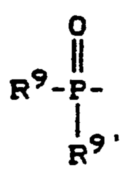

bedeutet,

worin
$R^7$ und $R^8$ gleich oder verschieden sind und geradkettiges oder verzweigtes Alkyl mit bis zu 8 Kohlenstoffatomen oder einen 6-gliedrigen Heterocyclus mit bis zu 3 Heteroatomen aus der Reihe Stickstoff, Sauerstoff oder Schwefel bedeuten,
$R^9$ und $R^{9'}$ gleich oder verschieden sind und Hydroxy oder Alkoxy mit bis zu 8 Kohlenstoffatomen bedeuten,
$R^4$ - einen Kohlenhydratrest mit 4 bis 8 Kohlenstoffatomen bedeutet, wobei die OH-Funktionen des Zuckers gegebenenfalls geschützt sind,
Q - Sauerstoff oder Schwefel bedeutet,
$R^5$ und $R^6$ gleich oder verschieden sind und Wasserstoff, geradkettiges oder verzweigtes Alkyl mit bis zu 8 Kohlenstoffatomen, Phenyl oder eine Aminoschutzgruppe bedeuten,
T - geradkettiges oder verzweigtes Alkenyl mit bis zu 8 Kohlenstoffatomen bedeutet, das gegebenenfalls durch Aryl mit 6 bis 10 Kohlenstoffatomen substituiert ist, das seinerseits durch

Halogen, Hydroxy oder Alkoxy mit bis zu 6 Kohlenstoffatomen substituiert sein kann,
A, B und D gleich oder verschieden sind und
- für eine direkte Bindung oder
- für einen Rest der Formel

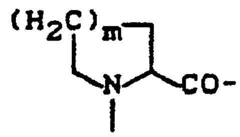

stehen
worin
m - die Zahl 1 oder 2 bedeutet,

oder
- für eine Gruppe der Formel

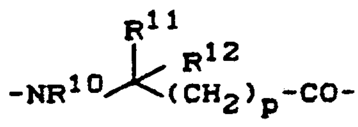

stehen
worin
p - die Zahl 0 oder 1 bedeutet,
$R^{10}$ Wasserstoff, geradkettiges oder verzweigtes Alkyl mit bis zu 6 Kohlenstoffatomen oder eine Aminoschutzgruppe bedeutet,
$R^{11}$ und $R^{12}$ gleich oder verschieden sind und
- Wasserstoff oder Cycloalkyl mit 3 bis 8 Kohlenstoffatomen bedeuten, oder
- einen 3- bis 8-gliedrigen Heterocyclus mit bis zu 4 Heteroatomen aus der Reihe Stickstoff, Sauerstoff oder Schwefel bedeuten,
- geradkettiges oder verzweigtes Alkyl mit bis zu 8 Kohlenstoffatomen bedeuten, das gegebenenfalls durch Alkylthio mit bis zu 6 Kohlenstoffatomen, Hydroxy, Mercapto, Guanidyl oder durch eine Gruppe der Formel $-NR^5R^6$ oder
$R^{13}$-OC- substituiert ist,

worin
$R^5$ und $R^6$ die oben angegebene Bedeutung haben

und
$R^{13}$ Hydroxy, Benzyloxy, Alkoxy mit bis zu 6 Kohlenstoffatomen oder die oben aufgeführte Gruppe $-NR^5R^6$ bedeutet,

oder das gegebenenfalls durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^5R^6$ substituiert sein kann,

worin
$R^5$ und $R^6$ die oben angegebene Bedeutung haben

oder das gegebenenfalls durch einen 5-oder 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,
L und M gleich oder verschieden sind und
- für eine direkte Bindung oder

- für eine Gruppe der Formel

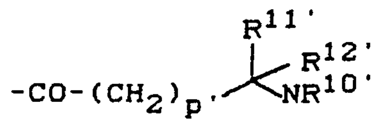

stehen
worin
p' , $R^{10'}$, $R^{11'}$ und $R^{12'}$ die oben angegebene Bedeutung von p, $R^{10}$, $R^{11}$ und $R^{12}$ haben,
in ihrer D- oder L-Form oder als D,L-Isomerengemisch,
$R^1$ und $R^2$ gleich oder verschieden sind und
- für geradkettiges oder verzweigtes Alkyl mit bis zu 8 Kohlenstoffatomen stehen, das gegebenenfalls durch Cycloalkyl mit 3 bis 8 Kohlenstoffatomen oder Aryl mit 6 bis 10 Kohlenstoffatomen substituiert ist,
Y - für eine Gruppe der Formel

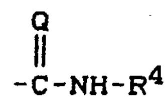

oder
$-CO-R^{14}$ steht,

worin
Q und $R^4$ die oben angegebene Bedeutung haben,
$R^{14}$ geradkettiges oder verzweigtes Alkyl mit bis zu 8 Kohlenstoffatomen bedeutet, das gegebenenfalls durch Pyridyl, Phenyl oder die Gruppe $-NR^{15}R^{16}$ substituiert ist,

worin
$R^{15}$ und $R^{16}$ entweder die oben angegebene Bedeutung von $R^5$ und $R^6$ haben und mit dieser gleich oder verschieden sind oder $R^{15}$ Wasserstoff bedeutet und
$R^{16}$ die Gruppe der Formel

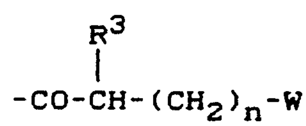

oder

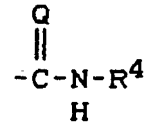

bedeutet worin
W, Q, n, $R^3$ und $R^4$ die oben angegebene Bedeutung haben
und deren physiologisch unbedenklichen Salze, mit der Maßgabe, daß X nur dann tert.-Butoxy bedeuten darf, wenn Y für die Gruppe Q

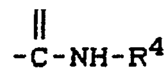

oder

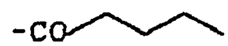

steht.

2. Verbindungen der allgemeinen Formel (I) gemäß Anspruch 1, in welcher
X - für Indolyl, tert.-Butoxy, Morpholino oder für eine Gruppe der Formel

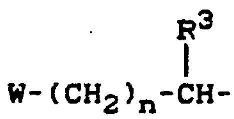 , 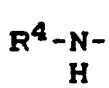 , 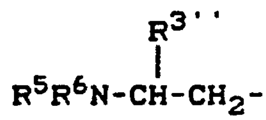

oder für T steht,

worin
$R^3$ und $R^{3'}$ gleich oder verschieden sind und geradkettiges oder verzweigtes Alkyl mit bis zu 6 Kohlenstoffatomen bedeuten, das gegebenenfalls durch Phenyl oder Naphthyl substituiert ist,
n - eine Zahl 1, 2 oder 3 bedeutet,
W - eine Gruppe der Formel
$R^7$-CO-, $R^8$-$SO_2$- oder

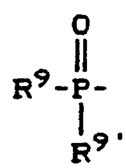

bedeutet
worin
$R^7$ und $R^8$ gleich oder verschieden sind und geradkettiges oder verzweigtes Alkyl mit bis zu 6 Kohlenstoffatomen oder Morpholino bedeuten,
$R^9$ und $R^{9'}$ gleich oder verschieden sind und Hydroxy oder Alkoxy mit bis zu 6 Kohlenstoffatomen bedeuten,
$R^4$ einen Pyranosylrest bedeutet, wobei die OH-Funktionen des Zuckers gegebenenfalls geschützt wird,
Q - Sauerstoff oder Schwefel bedeutet,
$R^5$ und $R^6$ gleich oder verschieden sind und Wasserstoff, geradkettiges oder verzweigtes Alkyl mit bis zu 6 Kohlenstoffatomen oder eine Aminoschutzgruppe bedeuten,
T - geradkettiges oder verzweigtes Alkyl oder Alkenyl mit bis zu 6 Kohlenstoffatomen bedeutet, das gegebenenfalls durch Phenyl substituiert ist, das seinerseits durch Fluor, Chlor, Hydroxy oder Alkoxy mit bis zu 4 Kohlenstoffatomen substituiert ist,
A, B und D gleich oder verschieden sind und
- für eine direkte Bindung oder
- für Prolin stehen, oder
- für eine Gruppe der Formel

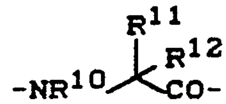

stehen

worin

$R^{10}$ Wasserstoff oder geradkettiges oder verzweigtes Alkyl mit bis zu 4 Kohlenstoffatomen bedeutet,

$R^{11}$ und $R^{12}$ gleich oder verschieden sind und Cyclopentyl, Cyclohexyl, Wasserstoff oder geradkettiges oder verzweigtes Alkyl mit bis zu 6 Kohlenstoffatomen bedeuten, das gegebenenfalls durch Naphthyl oder Phenyl substituiert ist, die ihrerseits durch Fluor, Chlor, Nitro oder Alkoxy mit bis zu 6 Kohlenstoffatomen substituiert sein können,

oder durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe substituiert sind,

L und M gleich oder verschieden sind und

- für eine direkte Bindung oder
- für eine Gruppe der Formel

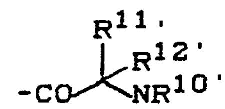

stehen

worin

$R^{10'}$, $R^{11'}$ und $R^{12'}$ die oben angegebene Bedeutung von $R^{10}$, $R^{11}$ und $R^{12}$ haben und mit dieser gleich oder verschieden sind,

in ihrer D- oder L-Form, oder als D,L-Isomerengemisch,

$R^1$ und $R^2$ gleich oder verschieden sind und für geradkettiges oder verzweigtes Alkyl mit bis zu 6 Kohlenstoffatomen stehen, das gegebenenfalls durch Cyclopropyl, Cyclopentyl, Cyclohexyl oder Phenyl substituiert ist,

Y - für eine Gruppe der Formel

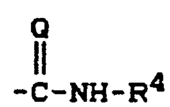

oder

-CO-$R^{14}$ steht,

worin

Q und $R^4$ die oben angegebene Bedeutung haben,

$R^{14}$ geradkettiges oder verzweigtes Alkyl mit bis zu 6 Kohlenstoffatomen bedeutet, das gegebenenfalls durch Pyridyl, Phenyl oder durch die Gruppe -$NR^{15}R^{16}$ substituiert ist,

worin

$R^{15}$ und $R^{16}$ entweder gleich oder verschieden sind und die oben angegebene Bedeutung von $R^5$ und $R^6$ haben und mit dieser gleich oder verschieden sind, oder

$R^{15}$ Wasserstoff bedeutet und

$R^{16}$ für eine Gruppe der Formel

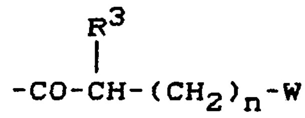

oder

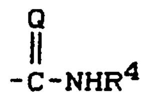

steht,
W, Q, n, $R^3$ und $R^4$ die oben angegebene Bedeutung haben
und deren physiologisch unbedenklichen Salze, mit der Maßgabe, daß X nur dann tert.-Butoxy bedeuten darf, wenn Y für die Gruppe Q

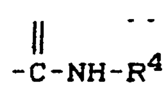

oder steht.

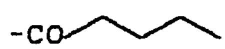

3. Verbindungen der allgemeinen Formel (I) gemäß Anspruch 1, in welcher
X - für Indolyl, tert.-Butoxy, Morpholino oder für eine Gruppe der Formel

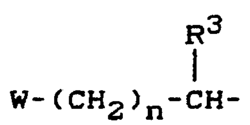 ,

$R^4$-NH- ,

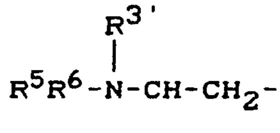

oder für T steht,

worin
$R^3$ und $R^{3'}$ gleich oder verschieden sind und geradkettiges oder verzweigtes Alkyl mit bis zu 4 Kohlenstoffatomen bedeuten, das gegebenenfalls durch Phenyl oder Naphthyl substituiert ist,
n - eine Zahl 1 oder 2 bedeutet,
W - eine Gruppe der Formel
$R^7$-CO- , $R^8$-$SO_2$- oder

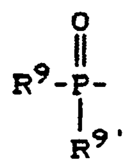

bedeutet,

worin
$R^7$ und $R^8$ gleich oder verschieden sind und geradkettiges oder verzweigtes Alkyl mit bis zu 4 Kohlenstoffatomen oder Morpholino bedeuten,
$R^9$ und $R^{9'}$ gleich oder verschieden sind und Hydroxy oder Alkoxy mit bis zu 4 Kohlenstoffatomen bedeuten,
$R^4$ - einen Rest der Formel

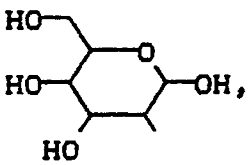
,
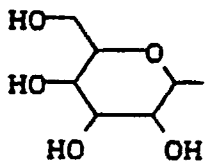
,
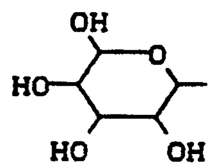
oder
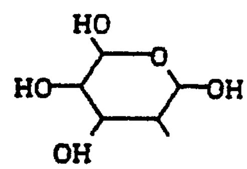

bedeutet
worin die OH-Funktionen gegebenenfalls durch Acetyl geschützt sind
Q - Sauerstoff oder Schwefel bedeutet,
$R^5$ und $R^6$ gleich oder verschieden sind und Wasserstoff, geradkettiges oder verzweigtes Alkyl mit bis zu 4 Kohlenstoffatomen oder eine Aminoschutzgruppe bedeuten,
T - den Rest der Formel

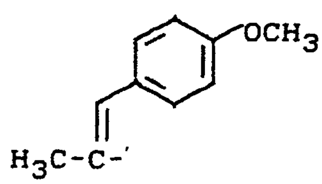

bedeutet,
A, B und D gleich oder verschieden sind und
- für eine direkte Bindung oder
- für Prolin stehen, oder
- für eine Gruppe der Formel

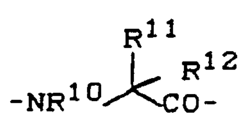

stehen,
worin
$R^{10}$ Wasserstoff oder Methyl bedeutet,
$R^{11}$ und $R^{12}$ gleich oder verschieden sind und Wasserstoff, Cyclopentyl oder geradkettiges oder verzweigtes Alkyl mit bis zu 4 Kohlenstoffatomen bedeuten, das gegebenenfalls durch Naphthyl oder Phenyl substituiert ist, die ihrerseits durch Fluor, Chlor oder Alkoxy mit bis zu 4 Kohlenstoffatomen substituiert sein können, oder

Alkyl durch Imidazolyl, Triazolyl, Pyridyl oder Pyrazolyl substituiert ist, wobei die -NH-Funktionen gegebenenfalls durch Methyl, Boc oder BOM geschützt sind,

L und M gleich oder verschieden sind und
- für eine direkte Bindung oder
- für eine Gruppe der Formel

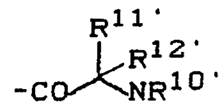

stehen,

worin

$R^{10'}$, $R^{11'}$ und $R^{12'}$ die oben angegebene Bedeutung von $R^{10}$, $R^{11}$ und $R^{12}$ haben und mit dieser gleich oder verschieden sind,

in ihrer D- oder L-Form, oder als D,L-Isomerengemisch,

$R^1$ und $R^2$ gleich oder verschieden sind und
- für geradkettiges oder verzweigtes Alkyl mit bis zu 4 Kohlenstoffatomen stehen, das gegebenenfalls durch Cyclohexyl oder Phenyl substituiert ist,

Y - für eine Gruppe der Formel

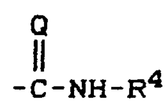

oder

$-CO-R^{14}$ steht,

worin

Q und $R^4$ die oben angegebene Bedeutung haben,

$R^{14}$ geradkettiges oder verzweigtes Alkyl mit bis zu 4 Kohlenstoffatomen bedeutet, das gegebenenfalls durch Pyridyl, Phenyl oder durch die Gruppe $-NR^{15}R^{16}$ substituiert ist,

worin

$R^{15}$ Wasserstoff bedeutet und

$R^{16}$ eine Gruppe der Formel

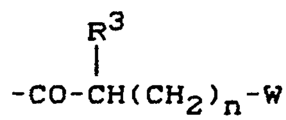

oder

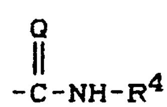

bedeutet,

worin

W, Q, n, $R^3$ und $R^4$ die oben angegebene Bedeutung haben

und deren physiologisch unbedenklichen Salze, mit der Maßgabe, daß X nur dann tert.-Butoxy bedeuten darf, wenn Y für die Gruppe Q

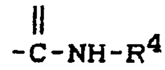

oder

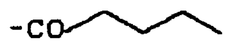

steht.

4. Verbindungen der allgemeinen Formel (I) gemäß Anspruch 1, zur Verwendung bei der Bekämpfung von Krankheiten.

5. Verfahren zur Herstellung von retroisosteren Dipeptiden der allgemeinen Formel (I)

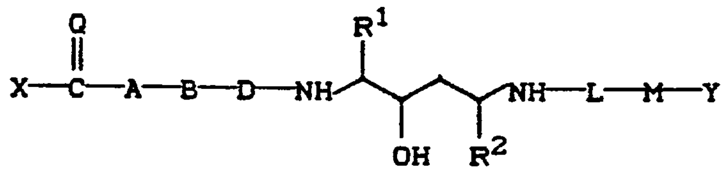

(I)

in welcher

X - für Indolyl, tert.-Butoxy, Morpholino oder für eine Gruppe der Formel

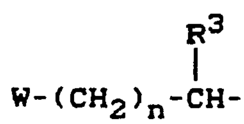

,

$R^4$-NH- ,

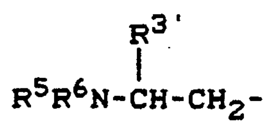

oder für T steht,

worin

$R^3$ und $R^{3'}$ gleich oder verschieden sind und geradkettiges oder verzweigtes Alkyl mit bis zu 8 Kohlenstoffatomen bedeuten, das gegebenenfalls durch Aryl mit 6 bis 10 Kohlenstoffatomen substituiert ist,

n - eine Zahl 1, 2, 3 oder 4 bedeutet,

W - eine Gruppe der Formel

$R^7$-CO-, $R^8$-$SO_2$- oder

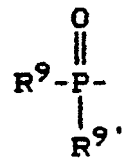

bedeutet,

worin

$R^7$ und $R^9$ gleich oder verschieden sind und geradkettiges oder verzweigtes Alkyl mit bis zu 8 Kohlenstoffatomen oder einen 6-gliedrigen Heterocyclus mit bis zu 3 Heteroatomen aus der Reihe Stickstoff, Sauerstoff oder Schwefel bedeuten,

$R^9$ und $R^{9'}$ gleich oder verschieden sind und Hydroxy oder Alkoxy mit bis zu 8 Kohlenstoffatomen bedeuten,

$R^4$ - einen Kohlenhydratrest mit 4 bis 8 Kohlenstoffatomen bedeutet, wobei die OH-Funktionen des Zuckers gegebenenfalls geschützt sind,

Q - Sauerstoff oder Schwefel bedeutet,

$R^5$ und $R^6$ gleich oder verschieden sind und Wasserstoff, geradkettiges oder verzweigtes Alkyl mit bis zu 8 Kohlenstoffatomen, Phenyl oder eine Aminoschutzgruppe bedeuten,

T - geradkettiges oder verzweigtes Alkenyl mit bis zu 8 Kohlenstoffatomen bedeutet, das gegebenenfalls durch Aryl mit 6 bis 10 Kohlenstoffatomen substituiert ist, das seinerseits durch Halogen, Hydroxy oder Alkoxy mit bis zu 6 Kohlenstoffatomen substituiert sein kann,m

A, B und D gleich oder verschieden sind und

- für eine direkte Bindung oder
- für einen Rest der Formel

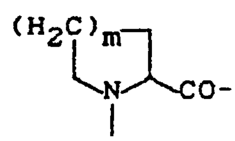

stehen

worin

m - die Zahl 1 oder 2 bedeutet,

oder

- für eine Gruppe der Formel

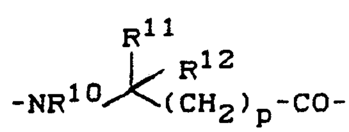

stehen

worin

p - die Zahl 0 oder 1 bedeutet,

$R^{10}$ Wasserstoff, geradkettiges oder verzweigtes Alkyl mit bis zu 6 Kohlenstoffatomen oder eine Aminoschutzgruppe bedeutet,

$R^{11}$ und $R^{12}$ gleich oder verschieden sind und

- Wasserstoff oder Cycloalkyl mit 3 bis 8 Kohlenstoffatomen bedeuten, oder
- einen 3- bis 8-gliedrigen Heterocyclus mit bis zu 4 Heteroatomen aus der Reihe Stickstoff, Sauerstoff oder Schwefel bedeuten,

- geradkettiges oder verzweigtes Alkyl mit bis zu 8 Kohlenstoffatomen bedeuten, das gegebenenfalls durch Alkylthio mit bis zu 6 Kohlenstoffatomen, Hydroxy, Mercapto, Guanidyl oder durch eine Gruppe der Formel $-NR^5R^6$ oder
$R^{13}$-OC- substituiert ist,

worin
$R^5$ und $R^6$ die oben angegebene Bedeutung haben

und
$R^{13}$ Hydroxy, Benzyloxy, Alkoxy mit bis zu 6 Kohlenstoffatomen oder die oben aufgeführte Gruppe $-NR^5R^6$ bedeutet,

oder das gegebenenfalls durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^5R^6$ substituiert sein kann,

worin

$R^5$ und $R^6$ die oben angegebene Bedeutung haben

oder das gegebenenfalls durch einen 5- oder 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,
L und M gleich oder verschieden sind und
- für eine direkte Bindung oder
- für eine Gruppe der Formel

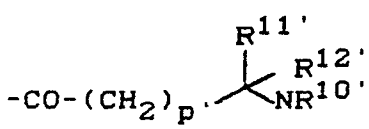

stehen
worin
$p'$, $R^{10'}$, $R^{11'}$ und $R^{12'}$ die oben angegebene Bedeutung von p, $R^{10}$, $R^{11}$ und $R^{12}$ haben,
in ihrer D- oder L-Form oder als D,L-Isomerengemisch,
$R^1$ und $R^2$ gleich oder verschieden sind und
- für geradkettiges oder verzweigtes Alkyl mit bis zu 8 Kohlenstoffatomen stehen, das gegebenenfalls durch Cycloalkyl mit 3 bis 8 Kohlenstoffatomen oder Aryl mit 6 bis 10 Kohlenstoffatomen substituiert ist,
Y - für eine Gruppe der Formel

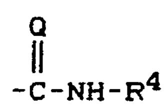

oder
$-CO-R^{14}$ steht,
worin
Q und $R^4$ die oben angegebene Bedeutung haben,
$R^{14}$ geradkettiges oder verzweigtes Alkyl mit bis zu 8 Kohlenstoffatomen bedeutet, das gegebenenfalls durch Pyridyl, Phenyl oder die Gruppe $-NR^{15}R^{16}$ substituiert ist,

worin
$R^{15}$ und $R^{16}$ entweder die oben angegebene Bedeutung von $R^5$ und $R^6$ haben und mit dieser gleich oder verschieden sind oder $R^{15}$ Wasserstoff bedeutet und

$R^{16}$ die Gruppe der Formel

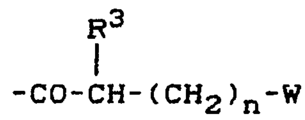

oder

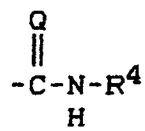

bedeutet
worin
W, Q, n, $R^3$ und $R^4$ die oben angegebene Bedeutung haben,
mit der Maßgabe, daß X nur dann tert.-Butoxy bedeuten darf, wenn Y für die Gruppe

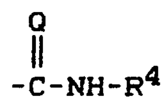

oder

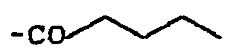

steht,

dadurch gekennzeichnet, daß man Verbindungen der allgemeinen Formel (II)

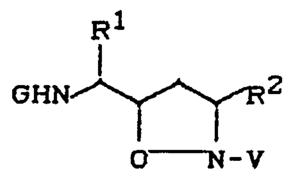

(II)

in welcher
$R^1$ und $R^2$ die oben angegebene Bedeutung haben,
G - für eine der oben aufgeführten Aminoschutzgruppen steht,
und
V - für einen hydrogenolytisch abspaltbaren Rest, wie beispielsweise Benzyl steht,

zunächst durch Hydrogenolyse unter Öffnung des Isoxazolidinrings zu den Aminoalkoholen der allgemeinen Formel (III)

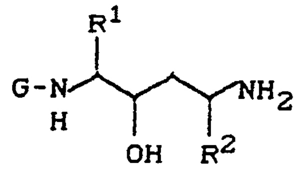 (III)

worin
G, Z, $R^1$ und $R^2$ die oben angegebene Bedeutung haben,

reduziert, gegebenenfalls anschließend mit Verbindungen der allgemeinen Formel (IV) oder (IVa)

$$HO—L'—M'—Y \quad (IV) \qquad HO-Y \quad (IVa)$$

in welcher
Y die oben angegebene Bedeutung hat
und

L' und M' die oben angegebene Bedeutung von L und M haben, aber nicht gleichzeitig für eine direkte Bindung stehen,

in inerten Lösemitteln, gegebenenfalls in Anwesenheit eines wasserentziehenden Hilfsstoffes und/oder einer Base kondensiert,
anschließend nach Abspaltung der Schutzgruppe G
nach bekannter Methode mit Verbindungen der allgemeinen Formel (V)

$$G'—B'—D'—OH \quad (V)$$

in welcher

B' und D' die oben angegebene Bedeutung von B und D haben, aber nicht gleichzeitig für eine direkte Bindung stehen

und

G' die oben angegebene Bedeutung von G hat und mit

dieser gleich oder verschieden ist,

umsetzt und in einem letzten Schritt nach Abspaltung der Schutzgruppe G' mit Verbindungen der Formel (VI)

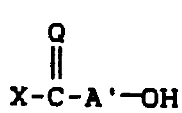 (VI)

in welcher

X und Q die oben angegebene Bedeutung haben

und

A' die oben angegebene Bedeutung von A hat, aber nicht für eine direkte Bindung steht,

gegebenenfalls in Anwesenheit einer Base umsetzt.

**6.** Verbindungen der allgemeinen Formel (II)

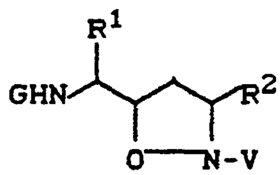

(II)

in welcher
$R^1$ und $R^2$ gleich oder verschieden sind und für geradkettiges oder verzweigtes Alkyl mit bis zu 8 Kohlenstoffatomen stehen, das gegebenenfalls durch Cycloalkyl mit 3 bis 8 Kohlenstoffatomen oder durch Aryl mit 6 bis 10 Kohlenstoffatomen substituiert ist,
G für eine Aminoschutzgruppe steht und
V für einen hydrogenolytisch abspaltbaren Rest wie z.B. Benzyl steht.

**7.** Verfahren zur Herstellung von Verbindungen der allgemeinen Formel (II)

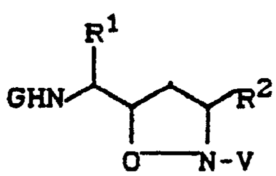

(II)

in welcher
$R^1$ und $R^2$ die oben angegebene Bedeutung haben,
G - für eine der oben aufgeführten Aminoschutzgruppen steht,

und
V - für einen hydrogenolytisch abspaltbaren Rest, wie beispielsweise Benzyl steht,

dadurch gekennzeichnet, daß man Verbindungen der allgemeinen Formel (VII)

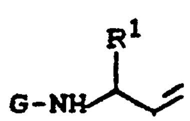

(VII)

in welcher

G die oben angegebene Bedeutung hat,

in einer Cycloadditionsreaktion mit Verbindungen der allgemeinen Formel (VIII)

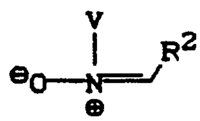

(VIII)

in welcher

V und $R^2$ die oben angegebene Bedeutung haben,

gegebenenfalls in Gegenwart von inerten organischen Lösungsmitteln umsetzt.

**8.** Arzneimittel, enthaltend mindestens eine Verbindung der allgemeinen Formel (I) gemäß Anspruch 1.

**9.** Verfahren zur Herstellung von Arzneimitteln, dadurch gekennzeichnet, daß man mindestens eine Verbindung der allgemeinen Formel (I) gemäß Anspruch 1 gegebenenfalls unter Verwendung üblicher Hilfs- und Trägerstoffe in eine geeignete Applikationsform überführt.

**10.** Verwendung von Verbindungen der allgemeinen Formel (I) gemäß Anspruch 1 bei der Herstellung von Arzneimitteln mit reninhibitorischer Wirkung.